(19) Europäisches Patentamt
European Patent Office
Office européen des brevets

(11) Veröffentlichungsnummer : **0 051 787**
**B1**

(12) # EUROPÄISCHE PATENTSCHRIFT

(45) Veröffentlichungstag der Patentschrift : **19.12.84**

(51) Int. Cl.³ : **C 07 D239/70**, C 08 G 18/20, C 08 G 59/40, C 09 D 3/58

(21) Anmeldenummer : **81108895.4**

(22) Anmeldetag : **24.10.81**

(54) **Neue Amidine und Amidin/Isocyanat-Addukte, Verfahren zu ihrer Herstellung und ihre Verwendung als Katalysatoren für die Härtung von Epoxidharzen.**

(30) Priorität : **06.11.80 DE 3041834**

(43) Veröffentlichungstag der Anmeldung : **19.05.82 Patentblatt 82/20**

(45) Bekanntmachung des Hinweises auf die Patenterteilung : **19.12.84 Patentblatt 84/51**

(84) Benannte Vertragsstaaten : **AT CH DE FR GB IT LI NL SE**

(56) Entgegenhaltungen :
**DE-A- 2 132 079**
**DE-A- 2 154 948**
**DE-A- 2 522 226**
**DE-A- 2 835 029**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Meyer, Rolf-Volker, Dr.**
**Buchheimer Strasse 23**
**D-4150 Krefeld (DE)**
Erfinder : **Kreuder, Hans-Joachim, Dr.**
**Doerperhofstrasse 35**
**D-4150 Krefeld (DE)**
Erfinder : **De Cleur, Eckhard, Dr.**
**Aubruchsgraben 14**
**D-4100 Duisburg 46 (DE)**

**EP 0 051 787 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue bicyclische Amidine und neue Amidin/Isocyanat-Addukte sowie Verfahren zu ihrer Herstellung und ihre Verwendung als Katalysatoren für die Härtung von Epoxidharzen, d. h. von wärmehärtbaren Massen auf Basis von Polyepoxiden und gegebenenfalls Polymeren, die Carboxylgruppen enthalten. Die Eigenschaften der erfindungsgemäßen Amidin/Isocyanat-Addukte kommen besonders beim Einsatz als Härter für Pulverlackbindemittel zur Geltung.

Pulverförmige Überzugsmittel, die durch Wirbelsintern, durch Flammspritzen oder nach dem elektrostatischen Pulversprühverfahren auf ein Substrat aufgebracht werden können, sind bekannt. Sie sollen bei mäßig hohen Temperaturen innerhalb möglichst kurzer Zeit zu vernetzten Beschichtungen eingebrannt werden können. Dabei ist zu beachten, daß einerseits beim Extrudieren der Mischung aus Bindemittel, Pigmenten, Füllstoffen und gegebenenfalls weiteren Hilfsmitteln bei Temperaturen von 80 bis 160 °C, vorzugsweise 90 bis 120 °C, keine Reaktion zwischen Harz und Härter stattfinden darf, andererseits aber ein Bedürfnis nach immer kürzeren Einbrennzeiten bei niedrigen Temperaturen, also nach erhöhter Reaktivität besteht.

Es ist zwar möglich, die Harzkomponente mit einer derart hohen Anzahl von Carboxylgruppen zu versehen, daß bei Einbrennbedingungen von 150-160 °C/30 Minuten eine vollständige Reaktion erzielt wird ; allerdings tritt aufgrund der hohen Säurezahl schon während des Extrusionsvorgangs eine unerwünschte Vorreaktion ein, die sich nur durch energisches Abkühlen des Extrudats in tolerierbaren Grenzen halten läßt. Auch die Lagerfähigkeit wird dadurch beeinträchtigt, daß selbst bei Raumtemperatur eine unerwünschte Reaktion abläuft, die dann den Verlauf des Pulverlacks und/oder die mechanischen Eigenschaften der Einbrennlackierung negativ beeinflußt.

Aus der DE-A-21 63 962 ist ein Pulverlackbindemittel bekannt, das aus einem Carboxylgruppenhaltigen Polyester und Triglycidylisocyanurat (vgl. Anspruch 4) besteht. Diese Bindemittel sollen zu wetterfesten (kreidungsbeständigen) Lackierungen führen. Im Normalfall liegen die Einbrennbedingungen derartiger Systeme für Schichtdicken von 40 bis 120 µm bei 200 °C/10 Minuten oder 160 °C/30 Minuten. Es bestand daher ein Bedürfnis, Pulverlacke bereitzustellen, die unter günstigeren Bedingungen gehärtet werden können, ohne daß Verlauf oder Lagerfähigkeit leiden.

Die DE-A-21 32 079 betrifft ein Verfahren zur Herstellung von ggf. C-alkylierten cyclischen Amidinen durch Umsetzung von Aldehyden mit Diaminoalkanen und elementarem Schwefel. Diese Amidine werden als Hilfsmittel und als Ausgangsmaterialien für die Herstellung von Pflanzenschutzmitteln und Farbstoffen empfohlen.

Die DE-A-21 54 948 betrifft ein Verfahren zur Herstellung ggf. substituierter cyclischer Amidine durch Umsetzung von Alkylendiaminen mit $\Delta^2$-Oxazolinen bei Temperaturen von 100 bis 240 °C. Eine Verwendung für die so hergestellten Amidine wird nicht angegeben.

Die DE-A-25 22 226 betrifft Vinyl-substituierte Imidazoline und Tetrahydropyrimidine, ein Verfahren zu ihrer Herstellung durch Umsetzung von Imidoestern mit Diaminen, anschließende Cyclisierung und Crackung. Die erhaltenen Amidine können zur Herstellung von Mono- oder Copolymerisaten eingesetzt werden.

In der DE-A-28 35 029 sind Tetrahydropyrimidin/Polyisocyanat-Addukte beschrieben, welche bei der Härtung von Überzugsmitteln gespalten werden, worauf dann die freigesetzten Polyisocyanate als Vernetzer wirken.

Keine der genannten Literaturstellen konnte die erfindungsgemäßen Amidine, ihre Umsetzungsprodukte mit Isocyanaten oder deren Verwendung als Katalysatoren für die Härtung von Epoxidharzen nahelegen.

Überraschenderweise wurde nun gefunden, daß Katalysatoren auf Basis bestimmter Isocyanatverkappter Amidine die Extrusion der Ausgangskomponenten zum gebrauchsfertigen Pulverlack unbeschadet überstehen und ihre Wirksamkeit erst im Zuge der endgültigen thermischen Aushärtung entfalten.

Im Sinne der Erfindung werden auch Guanidine als Unterklasse der Amidine betrachtet und deswegen ebenfalls als « Amidine » bezeichnet.

Gegenstand der Erfindung sind bicyclische Amidine der Formel

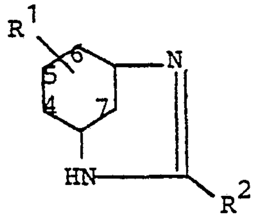

(I)

worin

$R^1$ ein Wasserstoffatom oder eine Alkylgruppe mit 1 bis 4 C-Atomen, vorzugsweise ein Wasserstoffatom oder eine Methylgruppe, und

$R^2$ ein Wasserstoffatom, eine Alkylgruppe mit 1 bis 18 C-Atomen, eine Cycloalkylgruppe mit 5 bis 14 C-Atomen, eine Aralkylgruppe mit 7 bis 16 C-Atomen oder eine Arylgruppe mit 6 bis 20 C-Atomen bedeuten.

Bevorzugte Amidine I sind folgende Verbindungen :

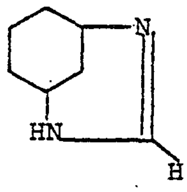

(II)

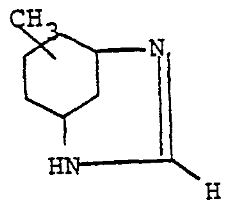

(III)

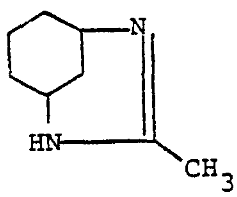

(IV)

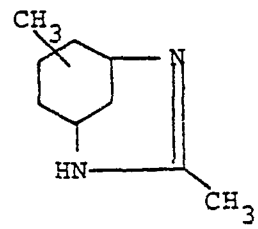

(V)

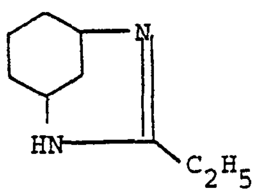

(VI)

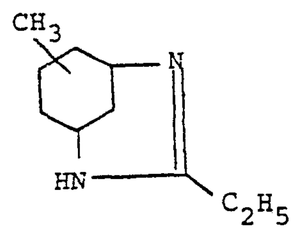

(VII)

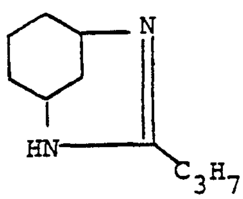

(VIII)

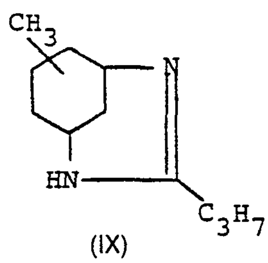

(IX)

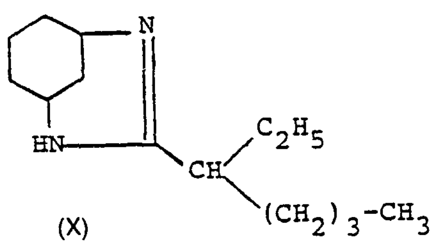

(X)

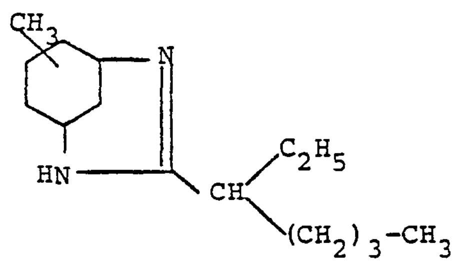

(XI)

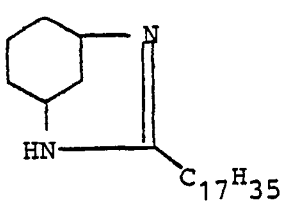

(XII)

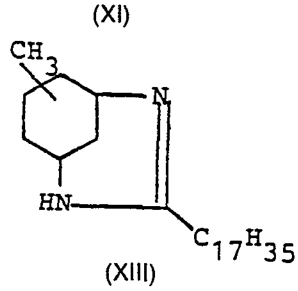

(XIII)

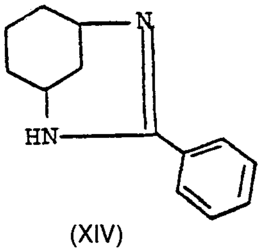

(XIV)

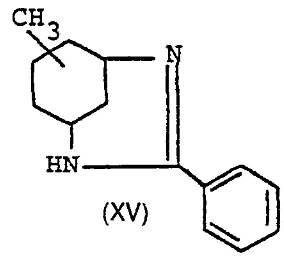

(XV)

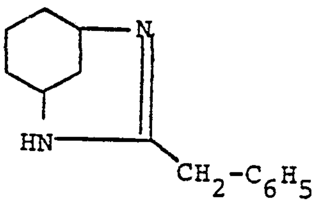

(XVI)

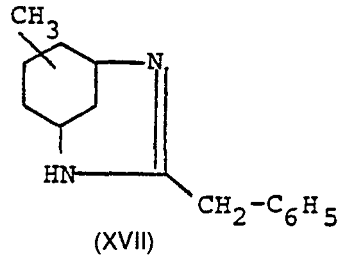

(XVII)

Die obigen mit ungeraden römischen Zahlen versehenen Formeln sind jeweils Abkürzungen für die vier verschiedenen Isomeren, in denen die Methylgruppe in 4-, 5-, 6- und 7-Position steht. Bevorzugt sind die 4- und 7-Methylderivate.

Die Amidine I lassen sich auf an sich bekannte Weise herstellen, vorzugsweise durch Reaktion der cycloaliphatischen Diamine XVIII mit — vorzugsweise gesättigten — Monocarbonsäuren XIX oder ihren reaktionsfähigen Derivaten XIX :

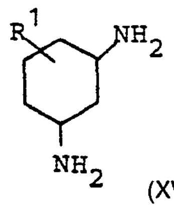

(XVIII)

$R^2-R^3$

(XIX)

worin

$R^1$ und $R^2$ die oben angegebene Bedeutung haben und
$R^3$ COCl, CN, $CO_2R^4$ und
$R^4$ ein Wasserstoffatom, $C_1$-$C_6$-Alkyl, Phenyl oder Benzyl bedeuten.

Neben 1,3-Diaminocyclohexan selbst sind bevorzugte Verbindungen XVIII insbesondere die Methyl-1,3-diaminocyclohexane, die durch Hydrierung der großtechnisch anfallenden Toluylendiamine entstehen. Die 2,4- und die 2,6-Diaminisomeren sowie ihre Gemische lassen sich gleichermaßen gut verwenden.

Bevorzugte Carbonsäuren XIX sind Ameisensäure, Essigsäure, Propionsäure, Buttersäure, 2-Ethylhexansäure, Stearinsäure, Benzoesäure und Phenylessigsäure.

Weiterer Gegenstand der Erfindung ist ein Verfahren zur Herstellung der Amidine I aus den Verbindungen XVIII und XIX, dadurch gekennzeichnet, daß man pro Mol Carbonsäure (derivat) gruppe der Verbindung XIX 1 bis 2,5, vorzugsweise 1 bis 1,5, Mol Diamin XVIII bei 150-280, vorzugsweise 200 bis 250 °C, 2 bis 10 Stunden lang miteinander reagieren läßt.

Der Überschuß an Diamin XVIII hat sich als vorteilhaft erwiesen, da er die Bildung des entsprechenden Diamids weniger begünstigt. In manchen Fällen ist es ratsam, einen Katalysator mitzuverwenden.

Bicyclische Amidine I sind zwar aus der DE-OS 27 22 514 (Formel Ic auf Seite 8) bekannt ; doch umfaßt die fragliche Formel 5-Ringe ebenso wie 6- und 7-Ringe und gibt, da es sich in dieser Literaturstelle um Reaktionsbeschleuniger für die Herstellung von Polyharnstoffen handelt, keinerlei Anregung, die erfindungsgemäßen Amidine I in Isocyanat-verkappter Form als Katalysatoren für die Härtung von Epoxidharzen einzusetzen.

Aus der DE-OS 27 31 335 sind Amidine des Imidazoltyps und ihre Verwendung zum Verkleben von in der Wärme härtbaren Einkomponenten-Epoxidsystemen bekannt. Für die Härtung von Pulverlacken auf Epoxidharzbasis weisen diese Imidazoline jedoch meist nicht die gewünschte Reaktivität auf.

In der DE-OS 27 51 805 werden Amidine vom Tetrahydropyrimidin-Typ und ihre Verwendung zur Blockierung von Polyisocyanaten beschrieben. Die blockierten Polyisocyanate sollen als Katalysatoren bei der anionischen Polymerisation von ε-Caprolactam sowie bei der Herstellung von Drahtlacken verwendet werden. Die erfindungsgemäßen Amidine I werden durch die DE-OS 27 51 805 weder vorbeschrieben noch nahegelegt.

Weiterer Gegenstand der Erfindung sind Additionsprodukte, erhältlich aus Polyisocyanaten — vorzugsweise solchen mit 4 bis 25, insbesondere 4 bis 16, C-Atomen und 2 bis 4, vorzugsweise 2, Isocyanatgruppen — und Amidinen der Struktur I.

Für die Umsetzung mit den Amidinen I zu Isocyanat-Additionsprodukten bevorzugte Polyisocyanate sind aliphatische, cycloaliphatische, araliphatische, d. h. arylsubstituierte aliphatische, und/oder aromatische Diisocyanate, wie sie beispielsweise in « Methoden der organischen Chemie » (Houben-Weyl), Bd. 14/2, 4. Aufl., Georg Thieme-Verlag, Stuttgart 1963, S. 61-70, und von W. Siefken, Liebigs Ann. Chem. *562*, 75-136, beschrieben werden, z. B. 1,2-Ethylendiisocyanat, 1,4-Tetramethylendiisocyanat, 1,6-Hexamethylendiisocyanat, 2,2,4- bzw. 2,4,4-Trimethyl-1,6-hexamethylendiisocyanat, 1,12-Dodecandiisocyanat, ω,ω'-Diisocyanatodipropylether, Cyclobutan-1,3-diisocyanat, Cyclohexan-1,3- und 4,4-diisocyanat, 2,2- und 2,6-Diisocyanato-1-methylcyclohexan, 3-Isocyanatomethyl-3,5,5-trimethylcyclohexylisocyanat (« Isophorondiisocyanat »), 2,5- und 3,5-Bis-(isocyanatomethyl)-1,4-methano-decahydronaphthalin, 1,5-, 2,5-, 1,6- und 2,6-Bis(isocyanatomethyl)-4,7-methano-hexahydroindan, 1,5-, 2,5-, 1,6- und 2,6-Bis-(isocyanato)-4,7-methano-hexahydroindan, Dicyclohexyl-2,4'- und -4,4'-diisocyanat, 2,4- und 2,6-Hexahydrotoluylendiisocyanat, Perhydro-2,4'- und -4,4'-diphenylmethan-diisocyanat, ω,ω', -Diisocyanato-1,4-diethylbenzol,1,3- und 1,4-Phenylendiisocyanat, 4,4'-Diisocyanato-diphenyl, 4,4'-Diisocyanato-3,3'-dichlordiphenyl, 4,4'-Diisocyanato-3,3'-dimethoxi-diphenyl, 4,4'-Diisocyanato-3,3'-dimethyl-diphenyl, 4,4'-Diisocyanato-3,3'-diphenyl-diphenyl, 2,4'- und 4,4'-Diisocyanato-diphenylmethan, Naphthylen-1,5-diisocyanat, Toluylendiisocyanate, 2,4- bzw. 2,6-Toluylen-diisocyanat, N,N'-(4,4'-Dimethyl-3,3'-diisocyanatodiphenyl)-uretdion, m-Xylylen-diisocyanat, aber auch die Triisocyanate wie 2,4,4'-Triisocyanatodiphenylether, 4,4',4''-Triisocyanatotriphenylmethan, Tris(4-isocyanatophenyl)-thiophosphat, sowie beliebige Gemische dieser Isomeren.

Besonders bevorzugt werden in der Regel die technisch leicht zugänglichen aliphatischen, cycloaliphatischen oder aromatischen Diisocyanate, insbesondere Hexamethylendiisocyanat, 3-Isocyanatomethyl-3,5,5-trimethylcyclohexylisocyanat und 2,4- bzw. 2,6-Toluylendiisocyanat sowie deren Isomerengemische.

Weitere bevorzugte Polyisocyanate zur Herstellung der Amidin/Polyisocyanat-Additionsprodukte sind aus den obengenannten Polyisocyanaten und mehrwertigen Alkoholen mit 2 bis 12 C-Atomen und 2 bis 6 OH-Gruppen erhältliche Präpolymere. Andere bevorzugte Polyisocyanate entstehen durch Selbstaddition einiger der obengenannten Polyisocyanate ; sie können neben freien Isocyanatgruppen noch Biuret-, Uretdion-, Uretonimin-, Isocyanurat-, Harnstoff- und/oder Allophanatgruppen enthalten.

Ein weiterer Gegenstand der Erfindung ist ein Verfahren zur Herstellung der oben erwähnten Additionsprodukte, dadurch gekennzeichnet, daß man die Amidine I und Polyisocyanate in einem Verhältnis, wonach auf eine NH-Gruppe der Amidine I 0,8 bis 3, vorzugsweise 0,95 bis 1,5, Isocyanatgruppen eingesetzt werden, bei 50 bis 150, vorzugsweise 80 bis 120 °C, miteinander umsetzt.

Es versteht sich von selbst, daß man in der Regel die bei der gewählten Reaktionstemperatur besser dosierbare Komponente dem vorgelegten erhitzten Reaktionspartner zugibt. So werden flüssige Polyisocyanate vorzugsweise dem flüssig vorgelegten Amidin I zugesetzt.

Die Reaktion kann in An- oder Abwesenheit inerter Lösungsmittel durchgeführt werden. Bevorzugte Lösungsmittel sind Ketone, wie Aceton, Methylethylketon, Methylisobutylketon, Cyclopentanon, Cyclohexanon ; Aromaten, wie Benzol, Toluol, Xylole, Chlorbenzol, Nitrobenzol ; cyclische Ether, wie Tetrahydrofuran, Dioxan ; Ester, wie Methylacetat, n-Butylacetat ; aliphatische Chlorkohlenwasserstoffe, wie Chloroform, Tetrachlorkohlenstoff ; sowie aprotische Lösungsmittel, wie Dimethylformamid, Dimethylacetamid, Dimethylsulfoxid.

Nach Beendigung der Reaktion wird gegebenenfalls das Lösungsmittel entfernt, die erhaltenen erfindungsgemäßen Amidin/Polyisocyanat-Additionsprodukte ausgetragen und in eine verarbeitungsgerechte Form überführt.

Die erfindungsgemäßen Amidin/Polyisocyanat-Addukte besitzen im allgemeinen ein konkretes Molekulargewicht, das sich aus Art und Menge der Ausgangskomponenten ergibt ; bei Einsatz eines Polyisocyanatgemisches mit Molekülen unterschiedlichen Molekulargewichts erhält man Additionsprodukte, die eine bestimmte Molekulargewichtsverteilung aufweisen. Sie besitzen meist ein mittleres Molekulargewicht $\bar{M}_n$ von 200 bis 3 000, vorzugsweise von 300 bis 1 000 (dampfdruckosmometrisch in Aceton bestimmt) und schmelzen im Bereich von 30 bis 220, vorzugsweise von 80 bis 160 °C (DIN 53 180). 53 180).

Ein weiterer Gegenstand der Erfindung sind Additionsprodukte, erhältlich aus Monoisocyanaten mit 2 bis 20 C-Atomen und Amidinen, die mindestens einmal die Struktur

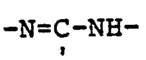 und/oder 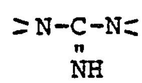

(XX) (XXI)

enthalten.

Bevorzugte Monoisocyanate sind Alkyl-, Cycloalkyl-, Aralkyl- und Arylisocyanate mit 2 bis 20 C-Atomen, wie z. B.

Methylisocyanat,
Ethylisocyanat,
Propylisocyanat,
Butylisocyanate,
2-Ethyl-hexylisocyanat,
6-Chlorhexylisocyanat,
Stearylisocyanat,
Phenylisocyanat und
Benzylisocyanat.

Vorzugsweise werden die weniger flüchtigen Isocyanate mit mindestens 6 C-Atomen verwendet, insbesondere 6-Chlorhexylisocyanat und Stearylisocyanat.

Besonders bevorzugte Amidine XX und XXI sind die obengenannten Amidine I ; weitere bevorzugte Amidine XX und XXI sind Imidazoline der Formel

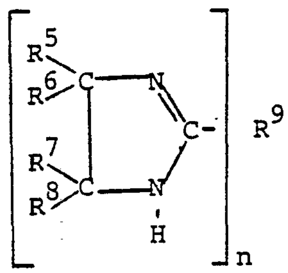

(XXII)

worin

$R^5$, $R^6$, $R^7$, $R^8$ ein Wasserstoffatom, einen Alkylrest mit 1 bis 6 C-Atomen, einen Cycloalkylrest mit 5 bis 10 C-Atomen, einen Aralkylrest mit 7 bis 12 C-Atomen, einen Arylrest mit 6 bis 15 C-Atomen oder einen heterocyclischen Rest mit 5 bis 10 C-Atomen und 1 bis 2 Sauerstoff- und/oder Stickstoff- und/oder Schwefelatomen und

$R^9$ einen Alkyl- oder Alkylenrest mit 1 bis 6 C-Atomen, einen Aryl- oder Arylenrest mit 6 bis 15 C-Atomen, die jeweils durch Alkyl-, Cycloalkyl-, Aralkyl-, Aryl- reste oder heterocyclische Reste (wie für $R^5$, $R^6$, $R^7$, $R^8$ definiert) substituiert sein können,

n 1 oder 2 und

$R^9$ für den Fall n = 1 auch ein Wasserstoffatom bedeuten.

Bevorzugte Imidazoline XXII sind z. B. 2-Phenylimidazolin, 2-Phenyl-4-methylimidazolin, 2-(m-Tolyl)-4-methyl-imidazolin, 2-(m-Pyridyl)-imidazolin, 1,4-Tetramethylen-bis-(4-methyl-imidazolin-2), 2-Methyl-imidazolin, 2,4-Dimethyl-imidazolin, 2-Ethyl-imidazolin, 2-Ethyl-4-methyl-imidazolin, 2-Benzyl-imidazolin, 2-(o-Tolyl)-imidazolin, 2-(p-Tolyl)-imidazolin, Tetramethylen-bis-imidazolin, 1,1,3-Trimethyl-1,4-tetramethylen-bis-imidazolin, 1,1,3-Trimethyl-1,4-tetramethylen-bis-4-methyl-imidazolin, 1,3,3-Trimethyl-1,4-tetramethylen-bis-4-methyl-imidazolin, 1,2-Phenylen-bis-imidazolin, 1,3-Phenylen-bis-4-methyl-imidazolin. Es können auch Gemische der Imidazolin-Derivate eingesetzt werden ; besonders bevorzugt werden 2-Phenyl-imidazolin oder 2-Methyl-imidazolin.

Weitere bevorzugte Amidine XX und XXI sind Tetrahydropyrimidine der Formel

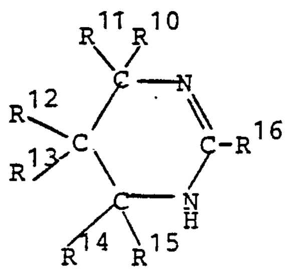

(XXIII)

worin

$R^{10}$-$R^{16}$ ein Wasserstoffatom, einen Alkylrest mit 1 bis 6 C-Atomen, einen Cycloalkylrest mit 5 bis 10 C-Atomen, einen Aralkylrest mit 7 bis 12 C-Atomen und einen Arylrest mit 6 bis 15 C-Atomen bedeuten, wobei auch zwei geminale oder vicinale Substituenten zusammen mit dem Kohlenstoffatom, an dem sie sitzen, einen cycloaliphatischen Ring mit 5 bis 6 C-Atomen bilden können.

Bevorzugte Tetrahydropyrimidine XXIII sind z. B. 2-Methyl-tetrahydropyrimidin, 2,4-, 2,5- und 2,6-Dimethyl-tetrahydropyrimidin, 2-Ethyltetrahydropyrimidin, 2-Ethyl-4-methyl-tetrahydropyrimidin, 2-Benzyl-tetrahydropyrimidin, 2-Phenyl-tetrahydropyrimidin, 2-Phenyl-4-methyl-, -5-methyl- und -6-methyl-tetrahydropyrimidin, 2,4-Diaza-3-phenyl-7,9,9- und -7,7,9-trimethylbicyclo(4,3,0)-nonen-2, 2,4-Diaza-3-methyl-7,9,9- und -7,7,9-trimethylbicyclo(4,3,0)-nonen-2 und Gemische dieser Tetrahydropyrimidine.

Weitere bevorzugte Amidine XX und XXI sind Verbindungen der Formel

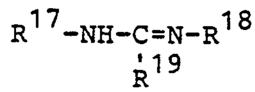

(XXIV)

worin

$R^{17}$, $R^{18}$, $R^{19}$ die oben für die Substituenten $R^5$ bis $R^8$ geschilderte Bedeutung haben.

$R^{17}$ und $R^{18}$ aber nicht gemeinsame Glieder eines Ringes sind.

Bevorzugte Amidine XXIV sind z. B. Formamidin, Acetamidin, Caproylamidin, Benzamidin, Benzyliminocaprolactam, n-Butyl- und tert.-Butyliminocaprolactam, N-Ethyl-N'-benzylbenzamidin, N-tert.-Butyl-N'-benzylacetamidin und N-n-Butylamino-N'-benzylacetamidin.

Weitere bevorzugte Amidine XX und XXI sind Guanidine der Formel

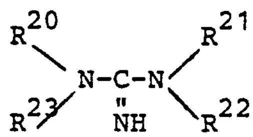

(XXV)

worin

$R^{20}$, $R^{21}$, $R^{22}$, $R^{23}$ ein Wasserstoffatom, einen $C_1$-$C_{12}$-Alkylrest, eine $C_5$-$C_{12}$-Cycloalkylrest, einen $C_7$-$C_{12}$-Aralkylrest, einen $C_6$-$C_{12}$-Arylrest oder einen $C_1$-$C_{12}$-Acylrest, vorzugsweise einen $C_1$-$C_4$-Alkylrest und einen Phenylrest bedeuten.

Bevorzugte Guanidine XXV sind z. B.

N-Methyl-guanidin,
N-Ethyl-guanidin,
N-Butyl-guanidin,
N-Methyl-N'-ethyl-guanidin,
N,N'-Dimethyl-guanidin,
N,N'-Diethyl-guanidin,
N-Methyl-N'-isopropyl-guanidin,
N,N'-Dibutyl-guanidin,
N,N,N'-Trimethyl-guanidin,
N,N,N',N'-Tetramethyl-guanidin,
N,N,N',N'-Tetraethyl-guanidin,
N-Phenyl-guanidin,
N,N'-Diphenyl-guanidin,
N,N'-Ditolyl-guanidin,
N-Formyl-guanidin und
N-Butyryl-guanidin.

Weiterer Gegenstand der Erfindung ist ein Verfahren zur Herstellung der genannten Amidin/Monoisocyanat-Additionsprodukte, dadurch gekennzeichnet, daß man die Amidine XX bzw. XXI und die Monoisocyanate mit 2 bis 20 C-Atomen in einem Verhältnis, wonach auf eine NH-Gruppe der Amidine XX bzw. XXI 0,8 bis 1,1, vorzugsweise ca. 1, Isocyanatgruppe, eingesetzt wird, bei 50 bis 130, vorzugsweise 80 bis 110 °C miteinander umsetzt.

Für die Mitverwendung eines Lösungsmittels gilt das oben für die Amidin/Polyisocyanat-Additionsprodukte Gesagte analog.

Ein weiterer Gegenstand der Erfindung ist die Verwendung der erfindungsgemäßen Amidin/Polyisocyanat- und Amidin/Monoisocyanat-Additionsprodukte als Katalysator für die Härtung von wärmehärtbaren Epoxidharzen, insbesondere für die Härtung pulverförmiger Überzugsmassen auf Basis von Polyepoxiden und gegebenenfalls Polymeren, die Carboxylgruppen enthalten.

Die erfindungsgemäßen Katalysatoren werden im allgemeinen in Mengen von 0,1 bis 5, vorzugsweise von 1 bis 2, Gew.-%, bezogen auf die Summe von Epoxidharz und dem gegebenenfalls vorhandenen sauren Polymeren, eingesetzt (Ausnahme s. S. 32). Die Zugabe wird vorzugsweise in einem Arbeitsgang bei der Herstellung des gebrauchsfertigen Pulverlacksystems durch Extrusion vorgenommen.

Die verwendbaren Polyepoxide sind feste, zumeist harzartige Stoffe, die im Bereich von 30 bis 140 °C, vorzugsweise zwischen 40 und 80 °C (bestimmt nach der Methode der Differentialthermoanalyse) schmelzen und die im Durchschnitt mehr als eine 1,2-Epoxygruppe pro Molekül enthalten.

Zum einen handelt es sich hierbei um Polyepoxidverbindungen auf Basis mehrwertiger Phenole, beispielsweise aus Brenzkatechin, Resorcin, Hydrochinon, aus 4,4'-Dihydroxidiphenylmethan, aus 4,4'-Dihydroxi-3,3'-dimethyl-diphenylmethan, aus 4,4'-Dihydroxidiphenyldimethylmethan (Bisphenol A), aus 4,4'-Dihydroxidiphenylmethylmethan, aus 4,4'-Dihydroxidiphenylcyclohexan, aus 4,4'-Dihydroxi-3,3'-dimethyldiphenylpropan, aus 4,4'-Dihydroxidiphenyl, aus 4,4'-Dihydroxidiphenylsulfon, aus Tris-(4-hydroxiphenyl)-methan, aus den Chlorierungs- und Bromierungsprodukten der vorstehend gennanten Diphenole, insbesondere aus Biphenol A ; aus Novolaken (d. h. aus Umsetzungsprodukten von ein- oder mehrwertigen Phenolen mit Aldehyden, insbesondere Formaldehyd, in Gegenwart saurer Katalysatoren), aus Diphenolen, die durch Veresterung von 2 Mol des Natriumsalzes einer aromatischen Oxicarbonsäure mit einem Mol eines Dihalogenalkans oder Dihalogendialkylethers erhalten wurden (vgl. britische Patentschrift 1 017 612), aus Polyphenolen, die durch Kondensation von Phenolen und langkettigen, mindestens 2 Halogenatome enthaltenden Halogenparaffinen erhalten wurden (vgl. britische Patentschrift 1 024 288).

Bevorzugt werden handelsübliche, feste Epoxidharze des Typs Diglycidylether des Bisphenols A (d. h. Umsetzungsprodukte von Bisphenol A mit Epichlorhydrin), deren Epoxidäquivalent zwischen 400 und 2 500 liegt, eingesetzt.

Weiter kommen Verbindungen wie (Poly)glycidylester der Formel

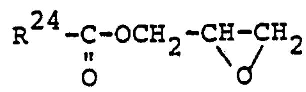

(XXVI)

in Frage, in denen $R^{24}$ ein geradkettiger oder verzweigtkettiger, gesättigter oder ungesättigter Kohlenwasserstoffrest mit 4-20 Kohlenstoffatomen oder ein gegebenenfalls substituierter Phenylrest ist.

Ferner können als erfindungsgemäß verwendbare Verbindungen Triglycidylisocyanurat und/oder dessen Oligomere und Triglycidylurazol sowie dessen Oligomere sowie Mischungen der genannten Stoffklassen eingesetzt werden.

Die carboxylgruppenhaltigen Polymeren können Polyesterpolycarbonsäuren sein, welche aus Polyolen und Polycarbonsäuren bzw. deren Derivaten hergestellt werden.

Die carboxylgruppenhaltigen Polymeren sollen einen Schmelz- und Erweichungsbereich (bestimmt durch Differentialthermoanalyse) von 20 °C-150 °C, vorzugsweise 50 °C-100 °C, und eine Säurezahl von 10-150, vorzugsweise 20-120, insbesondere von 30-50, haben. Die OH-Zahlen sollen vorzugsweise unter 20, insbesondere unter 10, liegen.

Die Veresterungsreaktion zum Aufbau der Polyestercarbonsäuren kann nach bekannten Verfahren durch Veresterung entsprechender Polycarbonsäuren und Polyole, insbesondere von Dicarbonsäuren und Dialkoholen oder durch Esterbildung aus geeigneten Derivaten dieser Alkohole und Carbonsäuren, wie z. B. der Anhydride, Säurechloride sowie auch mit Hydroxicarbonsäuren durchgeführt werden.

Durch den Einbau von mindestens trifunktionellen Polycarbonsäuren bzw. deren Anhydriden, wie Benzol-1,3,5-tricarbonsäure oder Trimellithsäureanhydrid werden besonders bevorzugte verzweigte, d. h. mindestens trisfunktionelle, Polyester-polycarbonsäuren erhalten.

Es können jedoch auch verzweigte Polyesterpolycarbonsäuren verwendet werden, welche durch den Einbau von vorzugsweise aliphatischen, mindestens drei Hydroxylgruppen tragenden Polyolen, wie Trimethylolpropan oder Glycerin, erhältlich sind.

Zur Herstellung der erfindungsgemäß zu verwendenden Polyester-polycarbonsäuren einzusetzende Polycarbonsäuren sind insbesondere solche der allgemeinen Formel

$$A\text{—}(COOH)_x \qquad \text{(XXVII)}$$

geeignet, wobei A eine Bindung oder einen x-wertigen, gegebenenfalls substituierten aliphatischen Rest mit vorzugsweise 1-20 C-Atomen, einen cycloaliphatischen Rest mit vorzugsweise 5-16 C-Atomen, einen aliphatisch-aromatischen Rest mit vorzugsweise 7-20 C-Atomen, einen aromatischen Rest mit vorzugsweise 6-15 C-Atomen, oder einen Heteroatome, wie N, O oder S, im Ring enthaltenden aromatischen oder cycloaliphatischen Rest mit 2-12 C-Atomen und x eine ganze Zahl von 2 bis 4, vorzugsweise 2 und 3, bedeuten.

Bevorzugte derartige Polycarbonsäuren sind beispielsweise :

Oxalsäure, Malonsäure, Bernsteinsäure, Glutarsäure, Adipin-, Trimethyladipin-, Azelain-, Sebacinsäure, Decandicarbonsäure, Dodecandicarbonsäure, Fumarsäure, Maleinsäure, Hexahydroterephthalsäure, Phthalsäure, Isophthalsäure, Terephthalsäure, Benzol-1,3,5-tricarbonsäure, Benzol-1,2,4-tricarbonsäure, Benzol-1,2,3-tricarbonsäure, Naphthalin-1,5-dicarbonsäure, Benzophenon-4,4'-dicarbonsäure, Diphenylsulfon-4,4'-dicarbonsäure, Butantetracarbonsäure, Tricarballylsäure, Ethylentetracarbonsäure, Pyromellithsäure, Benzol-1,2,3,4-tetracarbonsäure, Benzol-1,2,3,5-tetracarbonsäure sowie

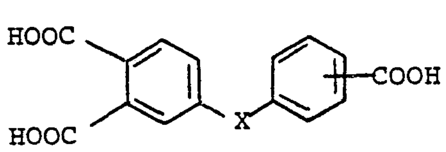 bzw. 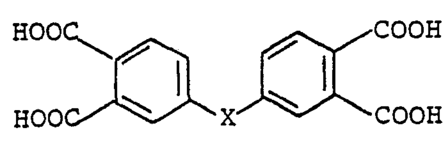

mit X = 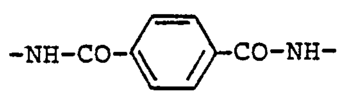

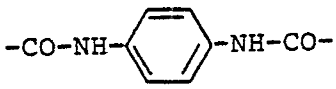

-O-, -S-, $-SO_2-$, -CO-

-N=N-, $-CH_2-$, $CH_3-\overset{|}{\underset{|}{C}}-CH_3$

Als Hydroxicarbonsäuren sind solche der Formel

$$(HOOC)-_{y}A-(OH)_{z} \qquad \text{(XXVIII)}$$

bevorzugt, in der A die oben angeführten Bedeutungen hat und y sowie z unabhängig voneinander eine ganze Zahl von 1 bis 3, vorzugsweise 1 oder 2, sein können.

Bevorzugte Beispiele sind Glykolsäure, Milchsäure, Mandelsäure, Apfelsäure, Zitronensäure, Weinsäure, 2-, 3- und 4-Hydroxibenzoesäure sowie Hydroxibenzoldicarbonsäuren.

Die zur Herstellung der Polyesterpolycarbonsäuren erforderlichen Polyole sind insbesondere solche der Formel

$$B-(OH)_{a} \qquad \text{(XXIX)}$$

wobei B einen a-wertigen aliphatischen Rest mit 2 bis 20 C-Atomen, einen cycloaliphatischen Rest mit 5 bis 16 C-Atomen, einen araliphatischen Rest mit 7 bis 20 C-Atomen, einen aromatischen Rest mit 6 bis 15 C-Atomen und einen N, O oder S enthaltenden heterocyclischen Rest mit 2 bis 12 C-Atomen und a eine ganze Zahl von 2 bis 6, vorzugsweise 2 und 3, bedeuten.

Als bevorzugte derartige Polyole seien aufgeführt :

Ethylenglykol, 1,2- und 1,3-Propandiol, 1,2-, 1,3-, 1,4- und 2,3-Butandiol, 1,5-Pentandiol, 2,2-Dimethyl-1,3-propandiol, 1,6- und 2,5-Hexandiol- 1,12-Dodecandiol, 1,12-Octadecandiol, 2,2,4- und 2,4,4-Trimethyl-1,6-hexandiol, Trimethylolpropan, Trimethylolethan, Gycerin, 1,2,6-Hexantriol, Pentaerythrit, Mannit, 1,4-Bis-hydroximethyl-cyclohexan, Cyclohexan-1,4-diol, 2,2-Bis-(4-hydroxicyclohexyl)-propan, Bis-(4-hydroxiphenyl)-methan, Bis-(4-hydroxiphenyl)-sulfon, 1,4-Bis-hydroximethyl-benzol, 1,4-Dihydroxibenzol, 2,2-Bis-(4-hydroxiphenyl)-propan, 1,3-Bis-hydroxialkylhydantoin, Trishydroxialkylisocyanurat und Trishydroxialkyltriazolidin-3,5-dion.

Weitere Polyole, die zur Herstellung der Polyesterpolycarbonsäuren geeignet sind, sind die durch Addition von gegebenenfalls substituierten Alkylenoxiden wie Ethylenoxid, Propylenoxid, Butylenoxid, Styroloxid an die oben erwähnten Polyole entstandenen Hydroxialkylether der Formel

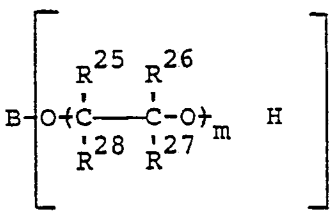

(XXX)

worin

B und a die oben angegebene Bedeutung haben, m für eine ganze Zahl von 1 bis 7 und $R^{25}$, $R^{26}$, $R^{27}$ und $R^{28}$ für Wasserstoffatom, einen gegebenenfalls mit Halogen substituierten aliphatischen $C_1$-$C_{10}$-Rest, cycloaliphatischen $C_4$-$C_8$-Rest, araliphatischen $C_7$-$C_{17}$-Rest oder einen gegebenenfalls mit Halogen, Alkyl und/oder Alkoxi substituierten aromatischen $C_6$-$C_{16}$-Rest stehen. Vorzugsweise bedeuten $R^{25}$, $R^{26}$, $R^{27}$ und $R^{28}$ Wasserstoff, einen Alkylrest mit 1 bis 4 C-Atomen, vorzugsweise Methyl, Ethyl oder einen gegebenenfalls mit Halogenatomen (Chlor, Brom), $C_1$-$C_4$-Alkylresten und/oder mit $C_1$-$C_4$-Alkoxigruppen substituierten $C_6$-$C_{12}$-Arylrest, insbesondere aber Phenyl.

Bevorzugte derartige Polyole sind beispielsweise : Diethylenglykol, Triethylenglykol, Dipropylenglykol, Tripropylenglykol, Dibutylenglykol, 1,4-Bis-/2-hydroxi-ethoxi/-cyclohexan, 1,4-Bis-/2-hydroxi-ethoxi-methan/-cyclohexan, 1,4-Bis-/2-hydroxi-ethoxi/-benzol, 4,4'-Bis-/2-hydroxiethoxi/-diphenylmethan, -diphenyl-propan-2, -diphenylether, -diphenylsulfon, -diphenylketon und -diphenylcyclohexan.

Selbstverständlich können die einzusetzenden Carbonsäuren bzw. Carbonsäurederivate und die zu verwendenden Polyole auch polymerer Natur sein. So können hier beispielsweise Bisbenzoldicarbonsäureester der Strukturformel

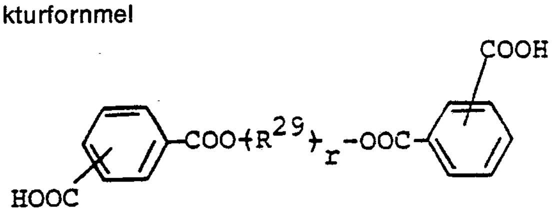

(XXXI)

und Bisalkandicarbonsäureester der Strukturformel

$$HOOC—(CH_2)_s———COO—(R^{30})_r———OOC—(CH_2)_t———COOH$$

eingesetzt werden.

$R^{29}$ und $R^{30}$ bedeuten darin einen mindestens zweiwertigen aromatischen Rest mit 6 bis 15 C-Atomen, einen araliphatischen Rest mit 7 bis 20 C-Atomen, einen gesättigten oder ungesättigten aliphatischen Rest mit 2 bis 20 C-Atomen, einen cycloaliphatischen Rest mit 5 bis 15 C-Atomen, der mit aromatischen ($C_6$-$C_{12}$), cycloaliphatischen ($C_4$-$C_{12}$) oder heterocyclischen ($C_2$-$C_{12}$) Ringsystemen kondensiert sowie über Ether-, Keto-, Ester- oder Sulfobrücken verbunden und der gegebenenfalls durch Halogen, Nitro- oder Alkoxigruppen mit 1 bis 20 C-Atomen substituiert sein kann. r steht für eine ganze Zahl von 1-20 ; s und t können gleich oder verschieden sein und bedeuten Null oder eine ganze Zahl von 1 bis 20.

Bevorzugte Beispiele für $(R^{29})_r$ bzw. $(R^{30})_r$ sind :

$-(CH_2)_2-$, $-(CH_2)_5-$, 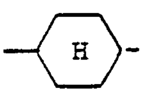, 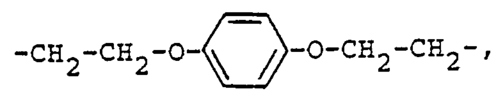,

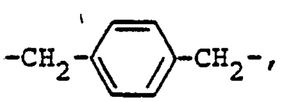, $-(CH_2)_2-O-(CH_2)_2-$, 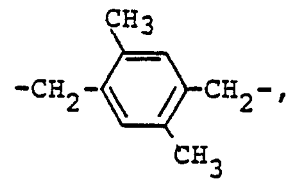,

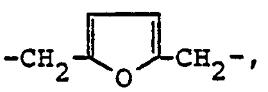, 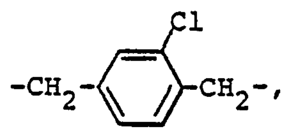, $-CH_2-CH=CH-CH_2-$,

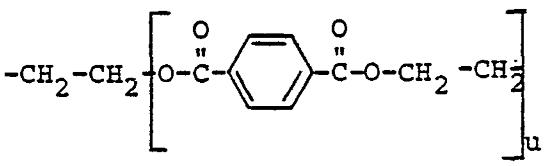 u = 1 - 7.

Die Herstellung dieser Polyesterpolycarbonsäuren erfolgt nach bekannten Verfahren in der Regel so, daß die Polycarbonsäuren und Polyole zusammen aufgeschmolzen werden und das freiwerdende Wasser eventuell durch Anlegen von Vakuum bzw. durch Ausblasen mit Stickstoff entfernt wird. Der Reaktionsverlauf kann dabei durch Titration der überschüssigen Carboxylgruppen verfolgt werden, so daß das Ende der Reaktion leicht bestimmt werden kann.

Selbstverständlich können auch Hydroxylgruppen enthaltende Polyester, die nach bekannten Verfahren aus Polycarbonsäuren, -anhydriden, -säurechloriden und/oder -alkylestern und Polyolen hergestellt werden, mit Polycarbonsäuren und -anhydriden zu den Polyester-polycarbonsäuren umgesetzt werden. Es ist natürlich auch möglich, solche hydroxylgruppenhaltige Polyester mit niedermolekularen sauren, d. h. carboxylgruppenhaltigen, Polyestern zu den Polyesterpolycarbonsäuren umzusetzen.

Werden verzweigte Polyesterpolycarbonsäuren benötigt, so kann deren Herstellung so erfolgen, daß alle Komponenten in Gegenwart eines mindestens trifunktionellen Alkohols bzw. einer mindestens trifunktionellen Polycarbonsäure nach den oben beschriebenen Verfahren durch Schmelzkondensation zu einem verzweigten Polyester kondensiert werden.

Es kann aber auch das mindestens trifunktionelle Polyol zusammen mit den Dicarbonsäuren bzw. -derivaten zu einem kurzkettigen Carboxylgruppen bzw. -derivate enthaltenden Polyester umgesetzt werden, welcher dann mit weiteren Diolen und Dicarbonsäuren zu den Polyesterpolycarbonsäuren kondensiert wird.

Selbstverständlich kann auch eine mindestens trifunktionelle Polycarbonsäure mit Diolen zu einem hydroxylgruppenhaltigen verzweigten kurzkettigen Polyester reagieren, welcher mit weiteren Diolen und Dicarbonsäuren zu den erfindungsgemäß zu verwendenden Polyesterpolycarbonsäuren weiter umgesetzt wird.

Verzweigte Polyesterpolycarbonsäuren sind natürlich auch erhältlich, wenn mindestens teilweise trifunktionelle Polycarbonsäuren mit dem oben beschriebenen hydroxylgruppenhaltigen Polyester zur Reaktion gebracht werden.

Eine weitere Gruppe von carboxylgruppenhaltigen Polymeren stellen die carboxylgruppenhaltigen Copolymerisate dar, welche aus copolymerisierten Einheiten von 2-25 Gewichtsteilen mindestens einer copolymerisierbaren, α-β-ethylenisch ungesättigten Carbonsäure mit 3-5 C-Atomen und 75-98 Gewichtsteilen mindestens eines weiteren copolymerisierbaren Monomeren bestehen. Die α,β-ethylenisch

ungesättigten Carbonsäuren können Monocarbonsäuren oder Dicarbonsäuren oder Halbester der Dicarbonsäuren mit 1-12 C-Atomen in der Alkoholkomponente sein.

Als bevorzugte copolymerisierbare Monomere seien genannt :

I. Maleinsäurediester und Ester der Acryl- oder Methacrylsäure mit aliphatischen $C_1$-$C_{12}$-, cycloaliphatischen $C_5$-$C_6$-, araliphatischen $C_7$-$C_8$-Monoalkoholen, beispielsweise Methylacrylat, Ethylacrylat, n-Propylacrylat, Isopropylacrylat, n-Butylacrylat, tert.-Butylacrylat, 2-Methylhexylacrylat, 2-Ethylhexylacrylat, Dodecylacrylat und die entsprechenden Methacrylsäureester und Maleinsäurediester ; Cyclopentylacrylat, Cyclohexylacrylat oder die entsprechenden Methacrylsäureester und Maleinsäurediester ; Benzylacrylat, β-Phenylethylacrylat, entsprechende Methacrylsäureester und Maleinsäurediester ;

II. Aromatische Vinyl- und Vinylidenverbindunge, beispielsweise Styrol, α-Methylstyrol, α-Methyl-p-isopropylstyrol, α-Methyl-m-isopropylstyrol, o-, p-Chlorstyrol, o-, p-Bromstyrol, kernsubstituierte Methylstyrole, p-tert.-Butyl-styrol oder deren Mischungen ;

III. Vinylester organischer Monocarbonsäuren, wobei die Säurekomponente 2-4 C-Atome enthält, wie Vinylacetat und Vinylpropionat ;

IV. monoolefinisch ungesättigte Halogenkohlenwasserstoffe, wie Vinylchlorid oder Vinylidenchlorid, vorzugsweise Vinylchlorid ;

V. Acrylnitril, Methacrylnitril, Acrylamid, Methacrylamid ;

VI. Vinylalkylether mit 1-4 C-Atomen in der Alkylgruppe, wie Vinylmethylether, Vinylethylether, Vinylpropylether, Vinylbutylether.

Bevorzugte carboxylgruppenhaltige Copolymerisate bestehen aus polymerisierten Einheiten von

a) 0-60 Gew.-% Styrol, α-Methylstyrol, o-, p-Chlor-styrol, o-, p-Bromstyrol, p-tert.-Butylstyrol oder deren Mischungen, vorzugsweise Styrol,

b) 0-98 Gew.-% Acrylsäureestern mit aliphatischen $C_1$-$C_8$-Alkoholresten oder Methacrylsäureestern mit aliphatischen $C_1$-$C_8$-Alkoholresten oder deren Mischungen

c) 2-25 Gew.-% Acrylsäure, Methacrylsäure, Itaconsäure-, Maleinsäure- und Furmarsäurehalbester mit 1-8 C-Atomen in der Alkoholkomponente oder deren Mischungen, vorzugsweise Acrylsäure und/oder Methacrylsäure,

wobei die Summe der Prozentgehalte von a bis c 100 beträgt. Anstelle von c) können auch 2-25 Gew.-% mindestens eines Hydroxylgruppen enthaltenden, olefinisch ungesättigten, copolymerisierbaren Monomeren wie Hydroxialkylester der Acryl-, Methacryl-, Malein-, Furmar- oder Itaconsäure mit 2 bis 4 C-Atomen im Alkoholrest im Copolymerisat vorhanden sein.

Bei der Verwendung der Hydroxylgruppen enthaltenden Monomeren werden Hydroxylgruppen enthaltende Copolymerisate erhalten, die durch Umsetzung mit Carbonsäureanhydriden wie z. B. Bernsteinsäureanhydrid in carboxylgruppenhaltige Copolymerisate übergeführt werden können.

Unter Copolymerisaten werden nicht nur Copolymerisate mit statistischer Verteilung der einpolymerisierten Monomeren oder Blockcopolymerisate verstanden, sondern auch Pfropfcopolymerisate, bei denen auf ein vorgebildetes Homo- und Copolymerisat Monomere aufgepfropft worden sind. Statistische Copolymerisate sind bevorzugt.

Die Herstellung der erfindungsgemäß zu verwendenden carboxylgruppenhaltigen Copolymerisate erfolgt nach bekannten Verfahren, wie Masse-, Lösungs-, Dispersions- und Perlpolymerisation, vorzugsweise durch Lösungs- oder Massepolymerisation. Derartige Verfahren sind beispielsweise in « Methoden der Organischen Chemie » (Houben-Weyl), 4. Auflage, Band 14/1, Seiten 24-556, Georg Thieme Verlag, Stuttgart, 1961, bzw. in der DE-OS 2 600 318 und DE-OS 1 965 740 beschrieben.

Die Mengen der einzelnen Pulverlack-Bindemittelkomponenten können weitgehend variiert werden.

Im Falle der ausschließlichen Verwendung (d. h. in Abwesenheit saurer Polyester) von Epoxidharzen des Typs Diglycidylether des Biphenols A mit einem Schmelzpunkt von 50 bis 120 °C und einem Epoxidäquivalent von 400 bis 2 000, können die praxisüblichen Härter, wie z. B. Dicyandiamid, Benzimidazol, Guanidine mit Vorteil durch die erfindungsgemäßen Amidin/Isocyanat-Additionsprodukte ersetzt werden, wobei man als Mischungsverhältnis von Harz/Härter in den Gewichtsgrenzen von 99 : 1 bis 85 : 15, vorzugsweise aber 97 : 3 bis 88 : 12, wählen kann.

Im Falle der Verwendung von Gemischen aus Epoxidharzen des Typs Diglycidylester des Bisphenols A und Carboxylpolyester richtet sich das Mischungsverhältnis nach der Säurezahl des Carboxylpolyesters. So wird z. B. üblicherweise bei einer Säurezahl von 30 bis 40 das Gewichtsverhältnis Epoxidharz/Carboxylpolyester 60 : 40 bis 80 : 20, vorzugsweise jedoch 70 : 30, betragen. Bei höheren Säurezahlen des Carboxylpolyesters, wie z. B. 40 bis 110, beläuft sich das praxisübliche Gewichtsverhältnis Epoxidharz/Carboxylpolyester auf 40 : 60 bis 60 : 40, vorzugsweise auf ca. 50 : 50.

Bei Verwendung von Carboxylpolyestern, die durch Zumischung von Triglycidylisocyanurat (TGIC) und/oder Triglycidylurazol (TGUZ) und/oder Glycidylestern gehärtet werden, ist das Harz/Härter-Verhältnis ebenfalls von der Säurezahl des Carboxylpolyesters abhängig. So beläuft sich das Gewichtsverhältnis von Carboxylpolyestern mit einer Säurezahl von 30 bis 40 mit Härtern wie TGIC und/oder TGUZ und/oder Glycidylestern auf 90 : 10 bis 95 : 5. Carboxylpolyester mit höheren Säurezahlen, wie z. B. 40-100, benötigen in der Regel einen höheren Härterzusatz, so daß das Gewichtsverhältnis von Carboxylpolyester zu Härtern wie TGIC und/oder TGUZ und/oder Glycidylestern 88 : 12 bis 92 : 8, beträgt.

Andere mögliche Bestandteile von Pulverlacken, wie z. B. Pigmente, Farbstoffe, Füllstoffe, Verlaufsmittel, Thixotropiermittel, Entlüftungsmittel, UV-Stabilisatoren und Oxidationsinhibitoren und Quencher

(Radikal-Fänger, wie z. B. N-alkylsubstituierte Piperidine) sowie Mattierungsmittel und solche Mittel, die die Oberflächenglätte verbessern, können innerhalb eines weiten Bereiches variiert werden.

Die Herstellung von Pulverlacken geschieht üblicherweise wie folgt : Die gewählten Bindemittel werden zusammen mit den erfindungsgemäßen Amidin/Isocyanat-Additionsverbindungen und gegebenenfalls weiteren Zusätzen zunächst gemischt und in der Schmelze homogenisiert. Dies kann in geeigneten Aggregaten, wie z. B. beheizbaren Knetern, vorzugsweise jedoch durch Extrudieren erfolgen, wobei die Extrusionstemperatur so zu wählen ist, daß ein Maximum an Scherkraft auf die Mischung einwirkt. Dabei sollte eine Temperaturobergrenze von 140 °C nicht überschritten werden.

Die extrudierte Masse wird nach Abkühlung auf Raumtemperatur und nach einer geeigneten Vorzerkleinerung zu einem Pulverlack gemahlen, wobei je nach dem Verwendungszweck mittlere Teilchengrößen von 40-70 μm, vorzugsweise jedoch ca. 50 μm, angestrebt werden. Ein eventuell vorhandener Grobkornanteil von Teilchengrößen von mehr als 100 μm wird durch Absieben entfernt.

Das Auftragen der so hergestellten Pulverlacke auf geeignete Substrate kann nach den bekannten Verfahren, wie z. B. durch elektrostatisches Pulversprühen, Wirbelsintern, elektrostatisches Wirbelsintern sowie im Flammspritzverfahren oder durch das Auftragen von wäßriger Suspension nach konventionellen oder elektrischen Verfahren erfolgen.

Nach dem Auftragen des Pulverlackes nach einem der genannten Verfahren werden die beschichteten Werkstücke zur Aushärtung auf Temperaturen von 140 °C oder höher erhitzt, wobei sich die Zeitdauer der Erhitzung im wesentlichen nach der Wärmekapazität des beschichteten Werkstückes oder dessen Temperatur vor der Beschichtung richtet.

Der erfindungsgemäße Vorteil beruht auf einer erheblichen Verkürzung der sogenannten Einbrennzeit und/oder Senkung der Einbrenntemperatur.

Im Falle der Verwendung der erfindungsgemäßen Isocyanatverkappten Amidine als Härterkomponente für Pulverlacke auf Basis von Epoxidharzen des Typs Diglycidylether von Bisphenol A liegen weitere Vorteile zum einen in der ausgezeichneten Verträglichkeit der Harzkomponente mit den in diesem Fall als Härter wirkenden erfindungsgemäßen Amidin/Isocyanat-Addukten, wodurch ein hoher Glanzgrad erzielt wird. Zum anderen wird die Herstellung des Pulverlackes erleichtert, weil eine unerwünschte Vorreaktion von Harz und Härter während des Extrusionsprozesses vermieden wird, da es bei der gegebenen Verweilzeit des Gemisches im Extruder und bei der benötigten Extrusionstemperatur in der Regel noch nicht zu einer Spaltung der Amidin/Isocyanat-Addukte kommt.

Ein weiterer Vorteil gegenüber der Anwendung bekannter Härter, wie z. B. Dicyandiamid, Aminen oder Guanidinen, ist in der Tatsache zu sehen, daß durch ihre Verwendung ein deutlichg besserer Verlauf erzielt werden kann.

Die gleichen Vorteile für die Verwendung der beschriebenen Amidin/Isocyanat-Addukte ergeben sich sinngemäß auch dann, wenn als Pulverlackbindemittel anstelle des Epoxidharzes vom Typ Diglycidylether des Bisphenols A eine Mischung desselben mit einem geeigneten Carboxylpolyesters benutzt wird oder wenn zur Herstellung von wetterbeständigen Beschichtungen als Bindemittel ein praxisüblicher Carboxylpolyester benutzt wird, der als Härterkomponente TGIC und/oder TGUZ und/oder geeignete Glycidylester enthält. In beiden Fällen sind die beschriebenen Amidin/Isocyanat-Addukte als latente Katalysatoren wirksam. Auch bei letzteren stellt sich das Problem der Senkung des Energiebedarfs beim Einbrennen durch Senkung der Einbrenntemperaturen und/oder Verkürzung der Einbrennzeiten. Das Problem war bisher nur durch solche Zusätze an Katalysatoren zu lösen, die eine Verschlechterung des Verlaufs der Beschichtungen und/oder eine Herabsetzung der Lagerfähigkeit des Beschichtungsmittels zur Folge hatten.

Die in den nachfolgenden Beispiele erwähnten Teile sind Gewichtsteile.

Beispiele

I. Herstellung des Amidins gemäß Formel A

A :

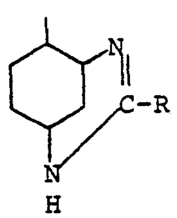

(Ca. 80 %)

+

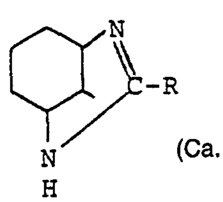

(Ca. 20 %)

Beispiel 1

Herstellung des Amidins der Formel A mit R = Phenyl

In einem 4 l Dreihalskolben, versehen mit Rührer, Thermometer und Claisenbrücke mit einem 1 l Vorlagekolben, werden 768 g (6 Mol) 2,4-Diamino (methyl-cyclohexan) (Isomerengemisch), im folgenden « PH-Tolamin » genannt, und 200 ml Wasser unter Rühren und $N_2$-Schutzgas vorgelegt. Dazu werden 732

g (6 Mol) Benzoesäure portionsweise innerhalb von 30 Minuten eingetragen. Die Temperatur steigt dabei auf 80 °C an. Sodann wird zügig auf 250 °C aufgeheizt, wobei Wasser und überschüssiges PH-Tolamin abdestillieren. Nach 4 Stunden bei 250 °C wird die Reaktion beendet. Das Destillat enthält 250 g Wasser und PH-Tolamin. Das verbleinbende Reaktionsprodukt wird bei ca. 0,3 mbar destilliert (bis 240 °C). Man erhält 733 g Rohprodukt, das zu gelblichen Kristallen erstarrt und 346 g Rückstand. Die Destillation des Rohproduktes ergibt folgende Fraktionen :

Vorlauf $Kp_{0,3\ mbar}$ 80-160 °C = 35 g (ca. 90 % PH-Tolamin)

Hauptlauf $Kp_{0,3\ mbar}$ 160-195 °C = 671 g Amidin, weiße Kristalle ; Fp 50-60 °C, , Rückstand : 27 g.

## Beispiel 2

Herstellung des Amidins der Formel A mit R = H

Analog Beispiel 1 werden 2 048 g (16 Mol) PH-Tolamin und 400 ml Wasser in 2 Stunden portionsweise mit 736 g (16 Mol) Ameisensäure versetzt. Die Temperatur steigt dabei auf 90 °C an. Dann wird zügig auf 250 °C aufgeheizt, wobei Wasser und überschüssiges PH-Tolamin abdestillieren. Nach 4 Stunden Nachrühren bei 250 °C is die Reaktion beendet. Das Destillat enthält 806 g Wasser und PH-Tolamin. Das verbleibende Reaktionsprodukt wird bei ca. 0,3 mbar (bis 200 °C) destilliert. Man erhält 806 g Rohprodukt (gelbes Öl, $Kp_{0,3}$ 120-170 °C). Der Rückstand beträgt 480 g. Die Destillation des Rohproduktes ergibt folgende Fraktionen :

Vorlauf $Kp_{0,3\ mbar}$ 35-107 °C = 139 g (90 % PH-Tolamin)

Hauptlauf $Kp_{0,3\ mbar}$ 107-130 °C = 595 g Öl

Rückstand 62 g. Gelbliches Öl, das allmählich zu einem Harz mit einem Erreichungsprodukt von 45-55 °C erstarrt.

## Beispiel 3

Herstellung des Adduktes aus Stearylisocyanat und dem Amidin des Beispiels 1

In einem 250 ml Dreihalskolben, versehen mit Rührer, Thermometer, Rückflußkühler und Tropftrichter, werden 49 g (0,2 Mol, bezogen auf Äquivalentgewicht) Amidin nach Beispiel 1 unter Rühren und $N_2$-Schutzgas bei 100 bis 120 °C mit 59 g (0,2 Mol) Stearylisocyanat in 20 Minuten portionsweise versetzt. Man erhält ein fast farbloses Harz mit einem Erweichungspunkt von ca. 35 °C.

## Beispiel 4

Herstellung des Adduktes aus Stearylisocyanat und dem Amidin des Beispiels 2

Analog Beispiel 3 werden 34,5 g (0,25 Mol) Amidin nach Beispiel 2 bei 100-120 °C mit 73,7 g (0,25 Mol) Stearylisocyanat in 20 Minuten portionsweise versetzt. Man erhält ein bräunliches Harz, das zu einem fast farblosen Pulver mit einem Erweichungspunkt von ca. 35 °C gemahlen wird.

## Beispiel 5

Herstellung des Adduktes aus Tetramethylguanidin und 6-Chlorhexylisocyanat

In einem 250 ml Dreihalskolben, versehen mit Rührer, Thermometer, Rückflußkühler und Tropftrichter werden 57,0 g (0,5 Mol) Tetramethylguanidin unter Rühren und $N_2$- Schutzgas bei 105-110 °C mit 80,5 g (0,5 Mol) 6-Chlorhexylisocyanat innerhalb von 20 Minuten portionsweise versetzt. Die leicht exotherme Reaktion ist nach weiteren 30 Minuten Nachrühren bei 110 °C beendet. Man erhält ein leicht gelbliches Harz mit einem Erweichungspunkt von ca. 35 °C. Der NCO-Gehalt beträgt 0,5 Gew.-%.

## Beispiel 6

Herstellung des Adduktes aus Hexamethylendiisocyanat und dem Amidin des Beispiels 1

Analog Beispiel 3 werden 122,5 g (0,5 Mol) Amidin nach Beispiel 1 bei 120-150 °C mit 42 g (0,25 Mol) Hexamethylendiisocyanat in 30 Minuten portionsweise versetzt. Man erhält hellgelbe Kristalle mit einem Schmelzpunkt von 79-86 °C.

## Beispiel 7

Herstellung des Adduktes aus Isophorondiisocyanat und dem Amidin des Beispiels 1

Analog Beispiel 3 werden 76,5 g (0,3 Mol) Amidin nach Beispiel 1 bei 120-130 °C mit 33,3 g (0,15 Mol) Isophorondiisocyanat in 30 Minuten portionsweise versetzt.

Man erhält hellgelbe Kristalle mit einem Schmelzpunkt von 108-113 °C.

### Beispiel 8

Herstellung des Adduktes aus Hexamethylendiisocyanat und dem Amidin des Beispiels 2

Analog Beispiel 3 werden 82,8 g (0,6 Mol) Amidin nach Beispiel 2 bei 130-150 °C mit 50,4 g (0,3 Mol) Hexamethylendiisocyanat in 30 Minuten portionsweise versetzt.

Man erhält hellgelbe Kristalle mit einem Schmelzpunkt von 85-90 °C.

### Beispiel 9

Herstellung des Adduktes aus Isophorondiisocyanat und dem Amidin des Beispiels 2

Analog Beispiel 3 werden 55,2 g (0,4 Mol) Amidin nach Beispiel 2 bei 130-185 °C mit 44,4 g (0,2 Mol) Isophorondiisocyanat in 30 Minuten portionsweise versetzt.

Man erhält hellgelbe Kristalle mit einem Schmp. von 155-160 °C. NCO-Gehalt : 0,2 Gew.-%.

## II. Herstellung der Pulverlacke

### Beispiel 10

38,5 Teile eines Carboxylpolyesters mit einer Säurezahl von 36, der aus Terephthalsäure (68,64 Teile), Isophthalsäure (10,27 Teile), Neopentylglykol (22,29 Teile), Ethylenglykol (13,29 Teile) und Glycerin (2,29 Teile) durch Schmelzkondensation unter Wasserabspaltung hergestellt wurde, werden mit 25,6 Teilen eines Epoxidharzes des Typs Diglycidylether des Bisphenols A mit einem Epoxidäquivalent von 850 und einer Glasübergangstemperatur von 59 °C (DTA) und mit 2,5 Teilen des erfindungsgemäßen Katalysators aus Beispiel 4 sowie mit 33,0 Teilen eines Weißpigmentes vom Typ eines hochbeständigen Titandioxid-Rutils und mit 0,4 Teilen eines Verlaufsmittels (Dichte 0,975 g/cm$^3$ ; Viskosität 23,3 Pa · s bei 20 °C) auf Basis eines Acrylat-Oligomeren, zunächst trocken gemischt. Diese Mischung wird auf einem Laborextruder der Firma Werner & Pfleiderer, Typ ZDSK 28, bei Temperaturen von 80-120 °C in der Schmelze dispergiert. Das Extrudat wird nach Abkühlung und Vorzerkleinerung auf einer Gebläsemühle des Typs 200 AS, Spiralstrahlmühle der Firma Alpine, Augsburg, auf eine durchschnittliche Korngröße von 50 µm zu einem Pulverlack gemahlen. Nach Absiebung des vorhandenen Grobkornanteils von Teilchengrößen oberhalb 100 µm wird der gebrauchsfertige Pulverlack auf doppelt dekapierte, entfettete Prüfbleche (Länge 165 mm, Breite 65 mm, Stärke 0,8 mm) mit einer elektrostatischen Sprühvorrichtung des Typs ESB, Sprühpistole Modell 50 458, bei einer Negativspannung von ca. 60 kV aufgesprüht. Die Bleche werden anschließend in einem Einbrennofen (Typ Kelvi Plast UL 350/S) 30 Minuten bei 140 °C eingebrannt. Die Beschichtungen werden nach den praxisüblichen Methoden auf ihre Eigenschaften geprüft (vgl. Tabelle I).

Bei diesen sowie bei den folgenden Versuchen wurden die lacktechnischen Eigenschaften nach folgenden Verfahren geprüft :

1) Gelierzeit des Pulverlackes nach DIN-Entwurf 55 990, Teil 8 ;

2) Tiefungswerte nach DIN 53 156 (nach Erichsen) ;

3) Schlagdeformation, rückwärtig in Anlehnung an ASTM G-14 mit einem Gewicht von 1 kg und einem Kugeldurchmesser von 1/2 Zoll ; Angabe in cm · kg (Fallhöhe · Gewicht) ;

4) Gitterschnittprüfung nach DIN 53 151 ;

5) Glanzgrad nach Gardner, DIN 67 530, 60°-Winkel,

6) Verlauf visuelle Abmusterung.

### Vergleichsversuch zu Beispiel 10

39,5 Teile des Carboxylpolyesters aus Beispiel 10 werden mit 26,3 Teilen des Epoxidharzes aus Beispiel 10 und mit 0,8 Teilen des unverkappten Katalysators aus Beispiel 2 sowie mit Pigment und Verlaufmittel wie in Beispiel 10 vermischt und analog Beispiel 10 zu einem Pulverlack verarbeitet, der auf Prüfbleche wie in Beispiel 10 aufgetragen und anschließend 30 Minuten bei 140 °C eingebrannt wird.

Prüfergebnisse siehe Tabelle I.

Beispiel 11

38,9 Teile des Carboxylpolyesters aus Beispiel 10 werden mit 25,8 Teilen des Epoxidharzes aus Beispiel 10 und mit 1,9 Teilen des Katalysators aus Beispiel 3 sowie mit Pigment und Verlaufmittel wie in Beispiel 10 vermischt und nach der in Beispiel 10 beschriebenen Methode zu einem Pulverlack verarbeitet, der auf Prüfbleche wie in Beispiel 10 aufgetragen und anschließend 30 Minuten bei 140 °C eingebrannt wird. Die Beschichtungen werden nach den beschriebenen Methoden auf ihre Eigenschaften geprüft (vgl. Tabelle I).

Vergleichsversuch zu Beispiel 11

39,5 Teile des Carboxylpolyesters aus Beispiel 10 werden mit 26,3 Teilen des Epoxidharzes aus Beispiel 10 und mit 0,8 Teilen des unverkappten Katalysators aus Beispiel 1 sowie mit Pigment und Verlaufmittel analog Beispiel 10 vermischt und zu einem Pulverlack verarbeitet, der auf Prüfbleche wie in Beispiel 10 aufgetragen und anschließend 30 Minuten bei 140 °C eingebrannt wird.
(Prüfergebnisse siehe Tabelle I).

Tabelle I

| | | Beispiel 10 | Vergleich zu Beispiel 10 | Beispiel 11 | Vergleich zu Beispiel 11 |
|---|---|---|---|---|---|
| Gelierzeit bei 180°C (in sec.) | | 64 - 65 | 53 - 54 | 60 - 61 | 52 - 53 |
| Tiefungswerte | Einbrenn-bedingung 30 Min/140°C | >10 | 9,8 | >10 | >10 |
| Schlagdeformation, rückwärtig | | 20 | 5 | 30 | 10 |
| Gitterschnitt | | Gt o/o | Gt o/o | Gt o/o | Gt o/o |
| Glanzgrad | | 92 | 86 | 92 | 88 |
| Verlauf | | besser als beim Vergleich | starke Struktur | besser als beim Vergleich | starke Struktur |

Beispiel 12

60,81 Teile eines handelsüblichen Carboxylpolyesters mit einer Säurezahl von 31 und einer Glasübergangstemperatur von 72 °C (DTA) werden mit 4,58 Teilen Triglycidylisocyanurat und mit 0,81 Teilen eines Weißpigments vom Typ eines hochbeständigen Titandioxid-Rutils und mit 0,7 Teilen des handelsüblichen Verlaufsmittels aus Beispiel 10 zunächst trocken gemischt. Diese Mischung wird auf einem Doppelschneckenextruder, Schneckendurchmesser 43 mm, bei 80 Umdrehungen/Min. und Temperaturen von 80-120 °C in der Schmelze dispergiert. Das Extrudat wird analog Beispiel 10 zu einem Pulverlack verarbeitet. Der so hergestellte Pulverlack wird auf Prüfbleche analog Beispiel 10 aufgesprüht und 10 Minuten bei 160 °C eingebrannt (Prüfergebnisse s. Tabelle II).

Vergleichsversuch zu Beispiel 12

61,32 Teile des Carboxylpolyesters aus Beispiel 12 werden mit 4,62 Teilen Triglycidylisocyanurat sowie 0,26 Teilen des Katalysators aus Beispiel 2 und im weiteren mit den gleichen Zusätzen wie in Beispiel 12 zu einem Pulverlack verarbeitet, auf die in Beispiel 10 beschriebenen Prüfbleche aufgesprüht und anschließend unter den gleichen Bedingungen wie in Beispiel 12 eingebrannt (Prüfergebnisse s. Tabelle II).

Beispiel 13

60,98 Teile des Carboxylpolyesters aus Beispiel 12 werden mit 4,59 Teilen Triglycidylisocyanurat

sowie 0,63 Teilen des Katalysators aus Beispiel 3 und mit 33,1 Teilen eines handelsüblichen, hochbeständigen Rutil-Titandioxid-Typs und mit 0,7 Teilen eines handelsüblichen Verlaufmittels wie in Beispiel 10 zu einem Pulverlack verarbeitet. Der so hergestellte Pulverlack wird auf Prüfbleche analog Beispiel 10 aufgesprüht und 10 Minuten bei 160 °C eingebrannt (Prüfergebnisse siehe Tabelle II).

Vergleichsversuch zu Beispiel 13

61,32 Teile des Carboxylpolyesters aus Beispiel 12 werden mit 4,62 Teilen Triglycidylisocyanurat sowie 0,26 Teilen des Katalysators aus Beispiel 1 und im weiteren mit den gleichen Zusätzen wie in Beispiel 13 zu einem Pulverlack verarbeitet und auf Prüfbleche aufgesprüht und anschließend unter den gleichen Bedingungen wie in Beispiel 13 eingebrannt (Prüfergebnisse s. Tabelle II).

Tabelle II

| | | Beispiel 12 | Vergleich zu Beispiel 12 | Beispiel 13 | Vergleich zu Beispiel 13 |
|---|---|---|---|---|---|
| Gelierzeit bei 180°C (sec) | | 53 - 54 | 66 - 67 | 58 - 59 | 57 - 59 |
| Tiefungswerte | Einbrennbedingungen 10 Min/160°C | 10 | 10 | 10 | 10 |
| Schlagdeformation, rückwärtig | | 50 | 40 | 60 | 90 |
| Gitterschnitt | | Gt o/o | Gt o/o | Gt o/o | Gt o/o |
| Glanzgrad | | 84 | 74 | 84 | 67 |
| Verlauf | | besser als beim Vergleich | starke Struktur | besser als beim Vergleich | starke Struktur |

Beispiel 14

60,9 Teile eines handelsüblichen Carboxylpolyesters mit einer Säurezahl von 31 und einer Glasübergangstemperatur von 66 °C (DTA) werden mit 4,6 Teilen Triglycidylisocyanurat sowie 0,7 Teilen des Katalysators aus Beispiel 5 und mit 33,1 Teilen eines handelsüblichen, hochbeständigen Rutil-Titandioxid-Typs und mit 0,7 Teilen eines handelsüblichen Verlaufmittels analog Beispiel 10 zu einem Pulverlack verarbeitet. Der so hergestellte Pulverlack wird auf Prüfbleche wie in Beispiel 10 aufgesprüht und 10 Minuten bei 160 °C eingebrannt (Prüfergebnisse siehe Tabelle III).

Vergleichsversuch zu Beispiel 14

61,31 Teile des Carboxylpolyesters aus Beispiel 14 werden mit 4,63 Teilen Triglycidylisocyanurat sowie 0,26 Teilen des handelsüblichen Produktes Tetramethylguanidin und im weiteren mit den gleichen Zusätzen wie in Beispiel 14 zu einem Pulverlack verarbeitet und auf Prüfbleche aufgesprüht und anschließend unter den gleichen Bedingungen wie in Beispiel 14 eingebrannt (Prüfergebnisse siehe Tabelle III).

(Siehe Tabelle III Seite 17 f.)

Tabelle III

| | | Beispiel 14 | Vergleich zu Beispiel 14 |
|---|---|---|---|
| Gelierzeit bei 180°C (sec) | | 52 - 53 | 47 - 49 |
| Tiefungswerte | Einbrennbedingungen 10 Min./160°C | 10 | >10 |
| Schlagdeformation, rückwärtig | | 70 | 50 |
| Gitterschnitt | | Gt o/o | Gt o/o |
| Glanzgrad | | 85 | 80 |
| Verlauf | | leichte Orangenschalenstruktur | kurznarbige Struktur |

Beispiel 15

39,2 Teile des Carboxylpolyesters aus Beispiel 10 werden mit 26,1 Teilen des Epoxidharzes aus Beispiel 10 und mit 1,3 Teilen des Katalysators aus Beispiel 8 sowie mit Pigment und Verlaufmittel wie in Beispiel 10 vermischt und nach der in Beispiel 10 beschriebenen Methode zu einem Pulverlack verarbeitet, der auf Prüfbleche wie in Beispiel 10 aufgetragen und anschließend 30 Minuten bei 140 °C eingebrannt wird (Prüfergebnisse siehe Tabelle IV).

Vergleichsversuch zu Beispiel 15

39,5 Teile des Carboxylpolyesters aus Beispiel 10 werden mit 26,3 Teilen des Epoxidharzes aus Beispiel 10 und mit 0,8 Teilen des unverkappten Katalysators aus Beispiel 2 sowie mit Pigment und Verlaufmittel wie in Beispiel 10 vermischt und nach der in Beispiel 10 beschriebenen Methode zu einem Pulverlack verarbeitet, der auf Prüfbleche wie in Beispiel 10 aufgetragen und anschließend 30 Minuten bei 140 °C eingebrannt wird (Prüfergebnisse siehe Tabelle IV).

Beispiel 16

39,4 Teile des Carboxylpolyesters aus Beispiel 10 werden mit 26,1 Teilen des Epoxidharzes aus Beispiel 10 und mit 1,1 Teilen des Katalysators aus Beispiel 6 sowie mit Pigment und Verlaufmittel wie in Beispiel 10 vermischt und nach der in Beispiel 10 beschriebenen Methode zu einem Pulverlack verarbeitet, der auf Prüfbleche wie in Beispiel 10 aufgetragen und anschließend 30 Minuten bei 140 °C eingebrannt wird (Prüfergebnisse siehe Tabelle IV).

Vergleichsversuch zu Beispiel 16

39,5 Teile des Carboxylpolyesters aus Beispiel 10 werden mit 26,3 Teilen des Epoxidharzes aus Beispiel 10 und mit 0,8 Teilen des unverkappten Katalysators aus Beispiel 1 sowie mit Pigment und Verlaufmittel wie in Beispiel 10 vermischt und nach der in Beispiel 10 beschriebenen Methode zu einem Pulverlack verarbeitet, der auf Prüfbleche wie in Beispiel 10 aufgetragen und anschließend 30 Minuten bei 140 °C eingebrannt wird (Prüfergebnisse siehe Tabelle IV).

(Siehe Tabelle IV Seite 18 f.)

Tabelle IV

| | | Beispiel 15 | Vergleich zu Beispiel 15 | Beispiel 16 | Vergleich zu Beispiel 16 |
|---|---|---|---|---|---|
| Gelierzeit bei 180°C (sec) | | 51 - 53 | 53 - 54 | 59 - 62 | 52 - 53 |
| | Einbrennbedingungen 30 Min/140°C | | | | |
| Tiefungswerte | | 10 | 9,8 | 10 | >10 |
| Schlagdeformation, rückwärtig | | 5 | 5 | 30 | 10 |
| Gitterschnitt | | Gt o/o | Gt o/o | Gt o/o | Gt o/o |
| Glanzgrad | | 89 | 86 | 94 | 88 |
| Verlauf | | besser als beim Vergleich | starke Struktur | besser als beim Vergleich | starke Struktur |

Beispiel 17

60,9 Teile des Carboxylpolyesters aus Beispiel 12 werden mit 4,6 Teilen Triglycidylisocyanurat und mit 0,7 Teilen des Katalysators aus Beispiel 9 sowie mit 33,1 Teilen eines Weißpigments vom Typ eines hochbeständigen Titandioxid-Rutils und mit 0,7 Teilen eines handelsüblichen Verlaufmittels analog Beispiel 10 zu einem Pulverlack verarbeitet. Der so hergestellte Pulverlack wird auf Prüfbleche wie in Beispiel 10 aufgesprüht und 10 Minuten bei 160 °C eingebrannt (Prüfergebnisse siehe Tabelle V).

Vergleichsversuch zu Beispiel 17

61,32 Teile des Carboxylpolyesters aus Beispiel 17 werden mit 4,62 Teilen Triglycidylisocyanurat sowie 0,26 Teilen des Katalysators aus Beispiel 2 und im weiteren mit den gleichen Zusätzen analog Beispiel 17 zu einem Pulverlack verarbeitet, auf die in Beispiel 10 beschriebenen Prüfbleche aufgesprüht und anschließend unter den gleichen Bedingungen wie in Beispiel 17 eingebrannt (Prüfergebnisse siehe Tabelle V).

Beispiel 18

60,90 Teile des Carboxylpolyesters aus Beispiel 14 werden mit 4,6 Teilen Triglycidylisocyanurat sowie 0,7 Teilen des Katalysators aus Beispiel 7 mit 33,1 Teilen eines handelsüblichen, hochbeständigen Rutil-Titandioxid-Typs und mit 0,7 Teilen eines handelsüblichen Verlaufmittels analog Beispiel 10 gemischt, extrudiert und zu einem Pulverlack verarbeitet. Der so hergestellte Pulverlack wird auf Prüfbleche wie im Beispiel 10 aufgesprüht und 10 Minuten bei 160 °C eingebrannt (Prüfergebnisse siehe Tabelle V).

Vergleichsversuch zu Beispiel 18

61,12 Teile des Carboxylpolyesters aus Beispiel 18 werden mit 4,62 Teilen des Polyepoxids aus Beispiel 18 sowie 0,46 Teilen des Katalysators aus Beispiel 1 und im weiteren mit den gleichen Zusätzen wie in Beispiel 18 zu einem Pulverlack verarbeitet, auf die in Beispiel 10 beschriebenen Prüfbleche aufgesprüht und anschließend unter den gleichen Bedingungen wie in Beispiel 18 eingebrannt (Prüfergebnisse siehe Tabelle V).

(Siehe Tabelle V Seite 19 f.)

Tabelle V

| | | Beispiel 17 | Vergleich zu Beispiel 17 | Beispiel 18 | Vergleich zu Beispiel 18 |
|---|---|---|---|---|---|
| Gelierzeit bei 180°C (sec) | | 54 - 55 | 63 - 65 | 76 - 79 | 43 - 45 |
| | Einbrennbedingungen 10 Min/160°C | | | | |
| Tiefungswerte | | 10 | 10 | 10 | 10 |
| Schlagdeformation, rückwärtig | | 130 | 10 | 80 | 70 |
| Gitterschnitt | | Gt o/o | Gt o/o | Gt o/o | Gt o/o |
| Glanzgrad | | 83 | 78 | 85 | 77 |
| Verlauf | | besser als beim Vergleich | starke Struktur | besser als beim Vergleich | starke Struktur |

**Ansprüche**

1. Bicyclische Amidine der Formel

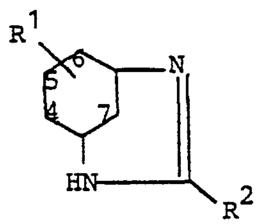

(I)

worin

$R^1$ ein Wasserstoffatom oder eine Alkylgruppe mit 1 bis 4 C-Atomen und

$R^2$ ein Wasserstoffatom, eine Alkylgruppe mit 1 bis 18 C-Atomen, eine Cycloalkylgruppe mit 5 bis 14 C-Atomen, eine Aralkylgruppe mit 7 bis 16 C-Atomen oder eine Arylgruppe mit 6 bis 20 C-Atomen bedeuten.

2. Amidine nach Anspruch 1, dadurch gekennzeichnet, daß $R^1$ für ein Wasserstoffatom oder eine Methylgruppe steht.

3. Amidine nach Ansprüchen 1 und 2, dadurch gekennzeichnet, daß sie aus den Strukturen

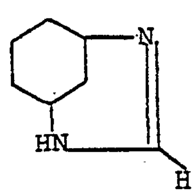

(II)

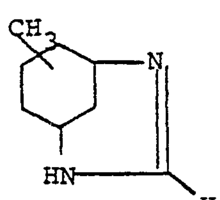

, (III)

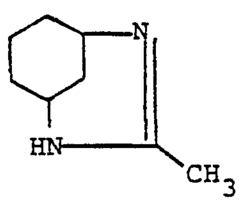

(IV)

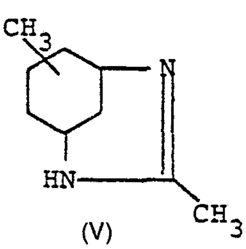

(V)

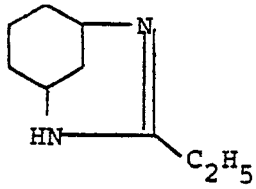

(VI)

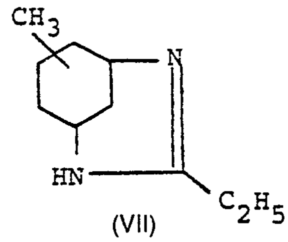

(VII)

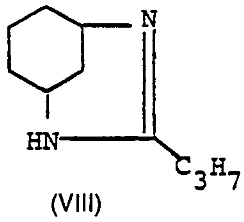

(VIII)

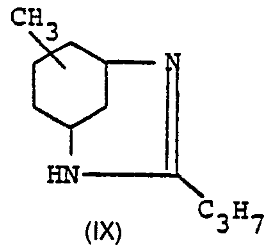

(IX)

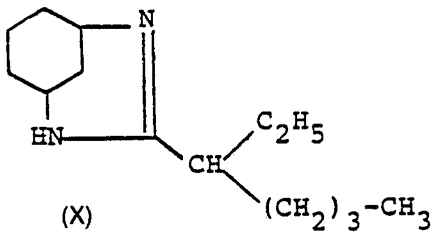

(X)

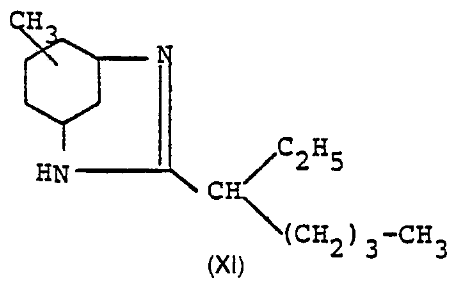

(XI)

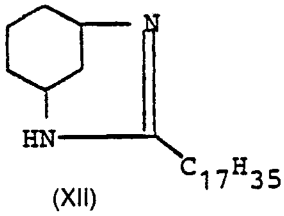

(XII)

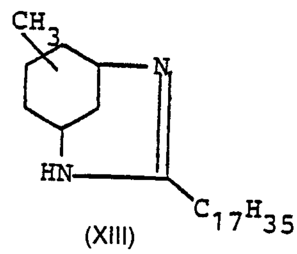

(XIII)

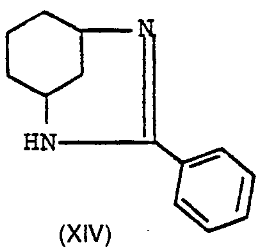

(XIV)

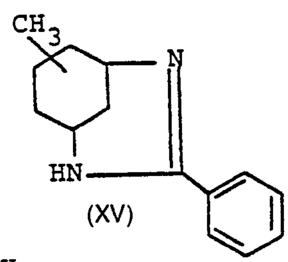

(XV)

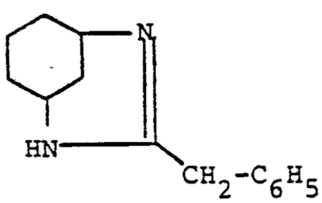

(XVI)

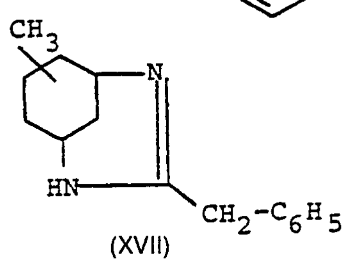

(XVII)

ausgewählt sind.

4. Verfahren zur Herstellung der Amidine nach Ansprüchen 1-3 aus den Verbindungen XVIII und XIX :

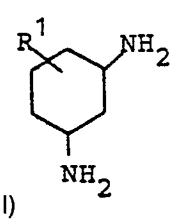

(XVIII)

$R^2-R^3$

(XIX)

worin

$R^1$ und $R^2$ die in Anspruch 1 angegebene Bedeutung haben und

$R^3$ COCl, CN, $CO_2R^4$ und

$R^4$ ein Wasserstoffatom, $C_1$-$C_6$-Alkyl, Phenyl oder Benzyl bedeuten,

dadurch gekennzeichnet, daß man pro Mol Carbonsäure (derivat) gruppe der Verbindung XIX 1 bis 2,5, vorzugsweise 1 bis 1,5, Mol Diamin XVIII bei 150-280, vorzugsweise 200 bis 250 °C 2 bis 10 Stunden lang miteinander reagieren läßt.

5. Reaktionsprodukte, erhältlich durch Umsetzung von Amidinen nach Ansprüchen 1-3 und Monoisocyanaten mit 2-20 C-Atomen oder Polyisocyanaten mit 4-25 C-Atomen, wobei

a) im Falle der Monoisocyanate das Verhältnis Amidin/Monoisocyanat derart gewählt wird, daß pro NH-Gruppe der Amidine nach Ansprüchen 1-3 0,8 bis 1,1 Isocyanatgruppen eingesetzt werden und die Umsetzung bei 50 bis 130, vorzugsweise 80 bis 110 °C erfolgt und

b) im Falle der Polyisocyanate das Verhältnis Amidin/Polyisocyanat derart gewählt wird, daß pro NH-Gruppe der Amidine nach Ansprüchen 1-3 0,8 bis 3, vorzugsweise 0,95 bis 1,5, Isocyanatgruppen eingesetzt werden, und die Umsetzung bei 50 bis 150, vorzugsweise 80 bis 120 °C erfolgt.

6. Verwendung der Reaktionsprodukte nach Anspruch 5 als Katalysator für die Härtung von wärmehärtbaren Epoxidharzen, insbesondere für die Härtung pulverförmiger überzugsmassen auf Basis von Polyepoxiden und gegebenenfalls Polymeren, die Carboxylgruppen enthalten.

7. Verwendung nach Anspruch 6, dadurch gekennzeichnet, daß die Katalysatoren in Mengen von 0,1 bis 5, vorzugsweise von 1 bis 2 Gew.-%, bezogen auf die Summe von Epoxidharz und dem gegebenenfalls vorhandenen sauren Polymeren, eingesetzt werden.

8. Verwendung nach Anspruch 6, dadurch gekennzeichnet, daß in Abwesenheit saurer Polyester das Epoxidharz ausschließlich Diglycidylether des Biphenols A mit einem Schmelzpunkt von 50-120 °C und einem Epoxidäquivalent von 400-2 000 ist und das Harz/Katalysator-Gewichtsverhältnis 99 : 1 bis 85 : 15 beträgt.

**Claims**

1. Bicyclic amidines of the formula

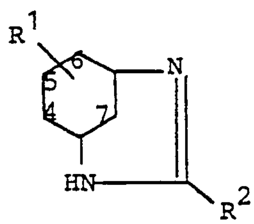

(I)

wherein

$R^1$ denotes a hydrogen atom or an alkyl group with 1 to 4 C atoms and

$R^2$ denotes a hydrogen atom, an alkyl group with 1 to 18 C atoms, a cycloalkyl group with 5 to 14 C atoms, an aralkyl group with 7 to 16 C atoms or an aryl group with 6 to 20 C atoms.

2. Amidines according to Claim 1, characterised in that $R^1$ represents a hydrogen atom or a methyl group.

3. Amidines according to Claims 1 and 2, characterised in that they are selected from the structures :

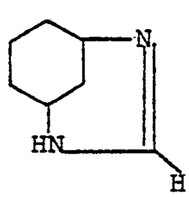

(II)

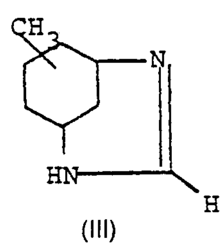

(III)

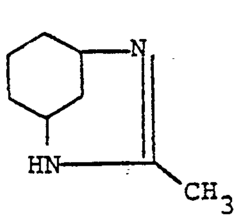

(IV)

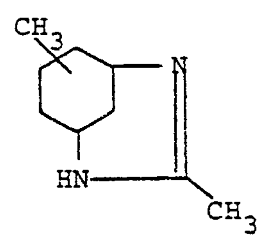

(V)

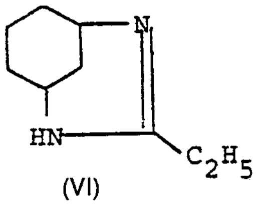

(VI)

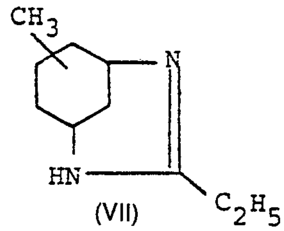

(VII)

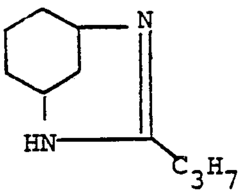

(VIII)

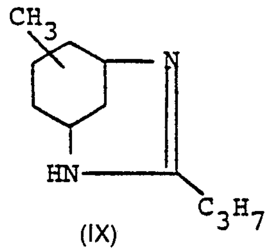

(IX)

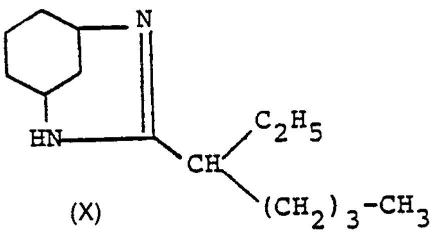

(X)

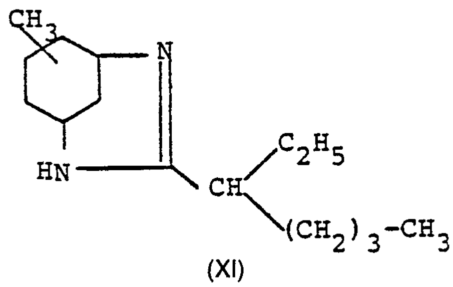

(XI)

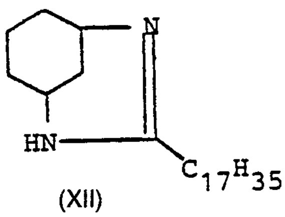

(XII)

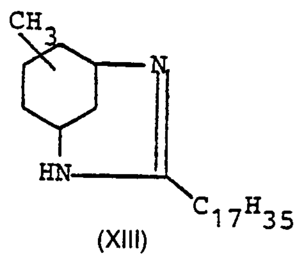

(XIII)

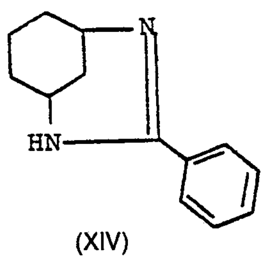

(XIV)

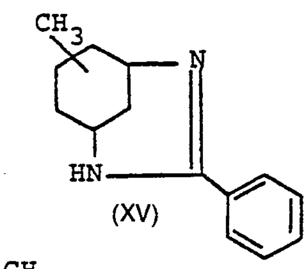

(XV)

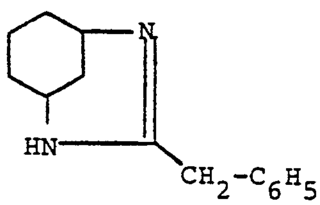

(XVI)

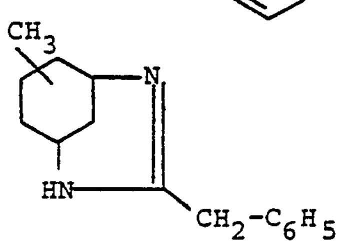

(XVII)

4. Process for the preparation of the amidines according to Claims 1-3 from the compounds XVIII and XIX :

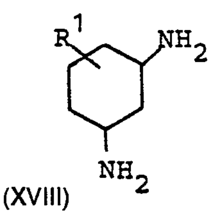

(XVIII)

$R^2-R^3$

(XIX)

wherein

$R^1$ and $R^2$ have the meaning given in Claim 1 and

$R^3$ denotes COCl, CN, $CO_2R^4$ and

$R^4$ denotes a hydrogen atom, $C_1$-$C_6$-alkyl, phenyl or benzyl,

characterised in that per mol of carboxylic acid (derivative) group of compound XIX, 1 to 2.5, preferably 1 to 1.5, mol of diamine XVIII are reacted together for 2 to 10 hours, at 150-280, preferably 200 to 250 °C.

5. Reaction products, obtainable by reacting amidines according to Claims 1-3 and monoisocyanates with 2-20 C atoms or polyisocyanates with 4-25 C atoms, wherein

a) in the case of the monoisocyanates the ratio of amidine/monoisocyanate is chosen such that per NH group of the amidines according to Claims 1-3, 0.8 to 1.1 isocyanate groups are used and the reaction is carried out at 50 to 130, preferably 80 to 110 °C and

b) in the case of the polyisocyanates the ratio of amidine/polyisocyanate is chosen such that per NH group of the amidines according to Claims 1-3, 0.8 to 3, preferably 0.95 to 1.5, isocyanate groups are used, and the reaction is carried out at 50 to 150, preferably 80 to 120 °C.

6. Use of the reaction products according to Claim 5 as a catalyst for the hardening of thermosetting epoxide resins, in particular for the hardening of pulverulent coating compositions based on polyepoxides and optionally polymers containing carboxyl groups.

7. Use according to Claim 6, characterised in that the catalysts are used in quantities of 0.1 to 5, preferably 1 to 2 % by weight, based on the sum of epoxide resin and the acid polymer optionally present.

8. Use according to Claim 6, characterised in that in the absence of acid polyesters the epoxide resin consists exclusively of diglycidyl ethers of bisphenol A with a melting point of 50-120 °C and an epoxide equivalent of 400-2,000 and the ratio by weight of resin/catalyst is 99 : 1 to 85 : 15.

**Revendications**

1. Amidines bicycliques de formule

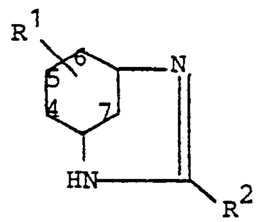

(I)

dans laquelle

$R^1$ est un atome d'hydrogène ou un groupe alkyle ayant 1 à 4 atomes de carbone et

$R^2$ est un atome d'hydrogène, un groupe alkyle ayant 1 à 18 atomes de carbone, un groupe cycloalkyle ayant 5 à 14 atomes de carbone, un groupe aralkyle ayant 7 à 16 atomes de carbone ou un groupe aryle ayant 6 à 20 atomes de carbone.

2. Amidines suivant la revendication 1, caractérisées en ce que $R^1$ représente un atome d'hydrogène ou un groupe méthyle.

3. Amidines suivant les revendications 1 et 2, caractérisées en ce qu'elles sont choisies entre les formules

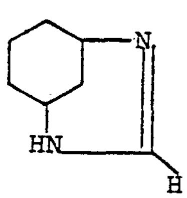

(II)

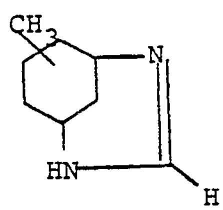

(III)

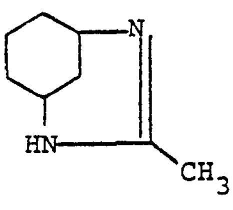

(IV)

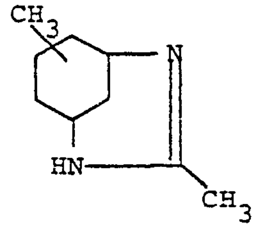

(V)

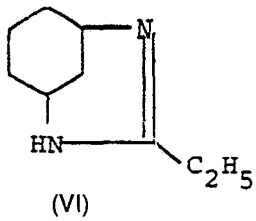

(VI)

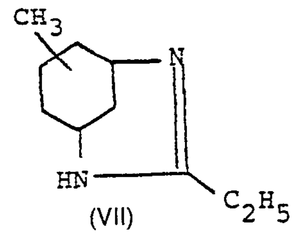

(VII)

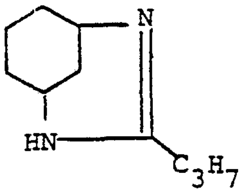

(VIII)

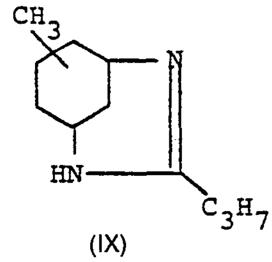

(IX)

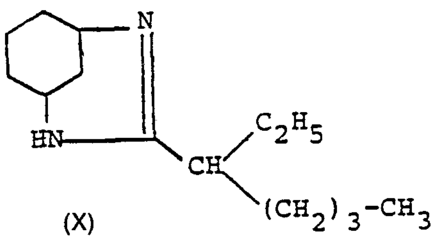

(X)

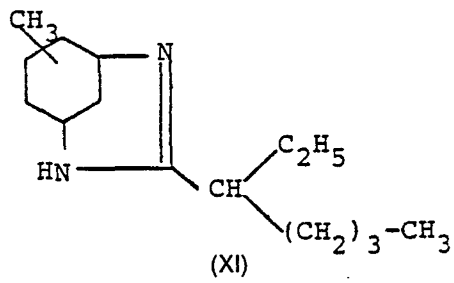

(XI)

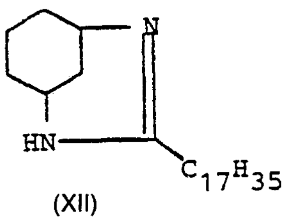

(XII)

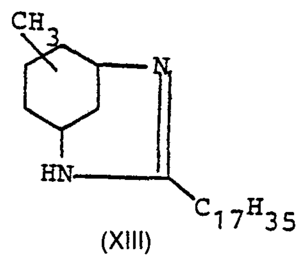

(XIII)

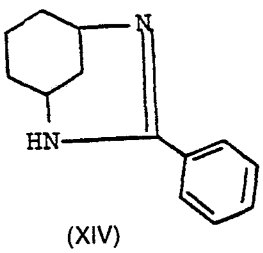

(XIV)

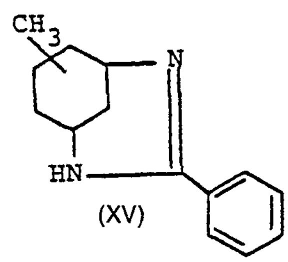

(XV)

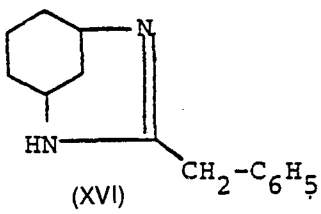

(XVI)

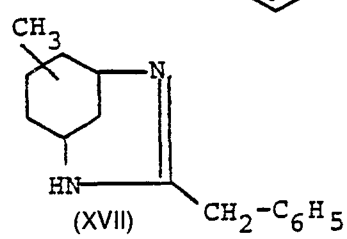

(XVII)

4. Procédé de production des amidines suivant les revendications 1-3 à partir des composés XVIII et XIX :

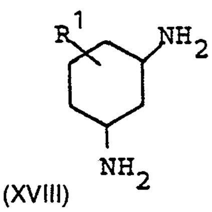

(XVIII)

$R^2-R^3$

(XIX)

dans lesquels

$R^1$ et $R^2$ ont la définition indiquée dans la revendication 1 et

$R^3$ représente COCl, CN, $CO_2R^4$ et

$R^4$ représente un atome d'hydrogène, un groupe alkyle en $C_1$ à $C_6$, phényle ou benzyle,

caractérisé en ce qu'on fait réagir ensemble, par mole de groupe (dérivé) acide carboxylique du composé XIX, 1 à 2,5, de préférence 1 à 1,5 mole de diamine XVIII à 150-280, de préférence 200-250 °C pendant 2 à 10 heures.

5. Produits de réaction pouvant être obtenus en faisant réagir des amidines suivant les revendications 1-3 et des mono-isocyanates ayant 2 à 20 atomes de carbone ou des polyisocyanates ayant 4 à 25 atomes de carbone, en sorte que,

a) dans les cas des mono-isocyanates, on choisit le rapport amidine/mono-isocyanate de manière à utiliser par groupe NH des amidines suivant les revendications 1 à 3, 0,8 à 1,1 groupe isocyanate et on conduit la réaction à 50-130 °C, de préférence à 80-110 °C et

b) dans le cas des polyisocyanates, on choisit le rapport amidine/polyisocyanate de manière à utiliser par groupe NH des amidines suivant les revendications 1 à 3, 0,8 à 3, de préférence 0,95 à 1,5 groupe isocyanate et on conduit la réaction à 50-150 °C, de préférence à 80-120 °C.

6. Utilisation des produits de réaction suivant la revendication 5, comme catalyseur pour le durcissement de résines époxy thermodurcissables, notamment pour le durcissement de matières de revêtement en poudre à base de polyépoxydes et, le cas échéant, de polymères qui contiennent des groupes carboxyle.

7. Utilisation suivant la revendication 6, caractérisée en ce que les catalyseurs sont utilisés en quantités de 0,1 à 5, de préférence de 1 à 2 % en poids par rapport à la somme de la résine époxy et du polymère acide éventuellement présent.

8. Utilisation suivant la revendication 6, caractérisée en ce que, en l'absence de polyesters acides, la résine époxy est exclusivement l'éther de diglycidyle du bisphénol A ayant un point de fusion de 50 à 120 °C et un équivalent d'époxyde de 400 à 2 000, et le rapport en poids de la résine au catalyseur va de 99 : 1 à 85 : 15.